# EUROPÄISCHE PATENTSCHRIFT

(11) **EP 0 677 107 B1**
(45) Veröffentlichungstag und Bekanntmachung des Hinweises auf die Patenterteilung: **06.08.1997**
(21) Anmeldenummer: 94900764.5
(22) Anmeldetag: 03.12.1993
(51) Int. Cl.: C12N 15/15, C07K 14/435, C12N 5/10, C12N 1/21, C12P 21/00, A61K 35/56, A61K 38/55

(54) **THROMBININHIBITOR AUS SPEICHEL VON PROTOSTOMIERN**
CLOTTING INHIBITOR MADE FROM PROTOSTOMIA SALIVA
INHIBITEUR DE THROMBINE PROVENANT DE SALIVE DE PROTOSTOMIENS

(30) Priorität: 04.12.1992 DE 4241659; 12.02.1993 DE 4304731; 17.08.1993 DE 4328336; 25.11.1993 DE 4340798
(43) Veröffentlichungstag der Anmeldung: 18.10.1995
(73) Patentinhaber: SCHERING AKTIENGESELLSCHAFT, 13342 Berlin (DE)
(72) Erfinder: NOESKE-JUNGBLUT, Christiane, D-14109 Berlin (DE); SCHLEUNING, Wolf-Dieter, D-13467 Berlin (DE); ALAGON, Alejandro, Cuernavaca Morelos 62271 (MX); POSSANI, Lourival, Cuernavaca Morelos 62271 (MX); CUEVAS-AGUIERRE, Delia, Cuernavaca Morelos 62271 (MX); DONNER, Peter, D-12169 Berlin (DE); HAENDLER, Bernard, D-10627 Berlin (DE); HECHLER, Ulrike, D-10627 Berlin (DE)
(86) Internationale Anmeldenummer: DE9301172
(87) Internationale Veröffentlichungsnummer: WO9413807

(56) Entgegenhaltungen:
- EP-A- 0 345 614
- WO-A-85/04418
- WO-A-91/04275

## Beschreibung

Die Erfindung betrifft Proteine, die Thrombininhibitoren aus Speichel von Protostomiern sind.

### STAND DER TECHNIK:

Thrombin hat eine Schlüsselfunktion bei der Thrombusbildung in Blutgefäßen. Es katalysiert die Spaltung von Fibrinogen zu Fibrin. Es führt zur Bildung von Blutgerinnseln. Weiterhin induziert es die Aggregation von Blutplättchen. Das Enzym ist in der Pathogenese verschiedener Krankheiten, wie z.B. arterieller und venöser Thrombose oder Arteriosklerose, involviert.

Deshalb ist der Einsatz eines Thrombininhibitors zur Therapie von Thrombosen erfolgversprechend. Die wichtigsten bisher bekannten Thrombininhibitoren sind Antithrombin III-Heparin und Hirudin. Antithrombin III ist ein im Plasma vorkommendes Protein mit einem Molekulargewicht von 58 kDa. Antithrombin III bindet zuerst an Heparin, das ein Polysaccharid ist. Dann bindet der Antithrombin III - Heparin - Komplex an Thrombin und inhibiert dieses Thrombin. Es entsteht ein sehr stabiler Komplex aus Thrombin und Antithrombin III und Antithrombin III wird von Thrombin gespalten. Neben Thrombin hemmt Antithrombin III auch andere Serinproteasen wie z.B. Faktor Xa (Pratt,C.W. und Church, F.C. (1991) "Antithrombin: Structure and Function", Seminars in Hematology **28:** 3-9).

Aus dem Blutegel *Hirudo medicinalis* wurde das Protein Hirudin isoliert. Es hat ein Molekulargewicht von etwa 7 kDa und bindet über ionische Wechselwirkung an das Thrombin. Es ist spezifisch für Thrombin ( Johnson, P.H. et al. (1989) "Biochemistry and Genetic engineering of hirudin", Seminars in Thrombosis and Hemostasis **15:** 302-315).

### BESCHREIBUNG DER ERFINDUNG:

Neben den zuvor erwähnten Thrombininhibitoren, die zum Stand der Technik zählen, sind weitere Inhibitoren notwendig, die einen anderen Wirkmechanismus, oder eine gesteigerte Aktivität besitzen.

Die Erfindung liefert natürliche oder synthetisch herstellbare Proteine, die Thrombininhibitoren sind und aus dem Speichel von Insekten, die Säugetierblut saugen, isolierbar sind.

Bevorzugt sind erfindungsgemäße Proteine, die aus der Raubwanze *Triatoma pallidipennis* isolierbar sind.

Die erfindungsgemäßen Proteine können natürlichen Ursprungs sein. Die Proteine werden gewonnen, indem der Speichel gereinigt wird. Ebenfalls ist esmöglich, die Proteine aus den Speicheldrüsen zu isolieren oder die das Protein synthetisierenden Zellen aus der Speicheldrüse zu nehmen und in Kultur zu halten. Die von dieser Zellkultur produzierten Überstände werden geerntet und aufgearbeitet. Der Zellüberstand wird gereinigt und die erfindungsgemäßen Proteine isoliert und angereichert. Alle Anreicherungsstufen der Isolierung und der Reinigung sind Teil der Erfindung. Bevorzugt sind die Anreicherungsstufen der Isolierung und Reinigung, bei denen die erfindungsgemäßen Proteine zu pharmazeutischen Zwecken verwendet werden können. So werden Reinigungen von 50 % des Thrombininhibitors bezogen auf das Gesamtprotein erzielt, bevorzugt sind 85 %, mehr bevorzugt 95 % und am meisten bevorzugt 99 % des Thrombininhibitors bezogen auf das Gesamtprotein.

Die Erfindung umfaßt nicht allein die Proteine, die von *Triatoma pallidipennis* isolierbar sind, sondern auch Proteine, die von anderen Insektenarten synthetisiert werden können. So sind neben den Proteine, die von *Triatoma pallidipennis* isolierbar sind, die zur Gruppe der bevorzugtesten zählen, weitere Proteine bevorzugt, die von *Triatoma infestans, Triatoma dimidiata, Triatoma maculata, Rhodnius prolixus, Panstrongylus megistus* und *Panstrongylus infestans* stammen.

Ebenso ist es möglich, die erfindungsgemäßen Proteine synthetisch herzustellen. Dazu zählt die Proteinsynthese nach J.M. SEWART and J.D. YOUNG, San Franzisco, 1969 and J. MEIENHOFER, Hormonal Proteins and Peptides Vol. 2 p 46, Academic Press (New York), 1973 und E. SCHODER and K. LUBKE, The Peptides, Vol. 1, Academic Press (New York) 1965. Zu den synthetisch hergestellten Proteinen zählen auch die rekombinanten Proteine, die nach bekannten Verfahren hergestellt werden. Je nach Wirtsorganismus können die erfindungsgemäßen Proteine glykosyliert oder, wenn sie in Prokaryonten synthetisiert werden, unglykosyliert sein.

Die Funktion der Inhibitoren ist in verschiedenen Testsystemen zu ermitteln. In den Beispielen 2 bis 4 und 9 sind gängige Testverfahren beschrieben.

Die erfindungsgemäßen Proteine sind in dem Speichel von Insekten, die Säugetierblut saugen, festzustellen. Diese Proteine werden üblicherweise von Zellen der Speicheldrüsen synthetisiert. Daher können die Proteine aus dem Speichel isoliert werden. Die erfindungsgemäßen Proteine sind nicht auf diese Herstellungsweise und Isolierung beschränkt. Vielmehr sind alle synthetisch hergestellten, erfindungsgemäßen Thrombininhibitoren mitumfaßt, die sich im dem Speichel nachweisen und daraus isolieren lassen.

### N-TERMINALE SEQUENZEN DES REIFEN PROTEINS

Die Erfindung umfaßt weiterhin ein natürliches oder synthetisch herstellbares Protein, das ein Thrombininhibitor ist und aus dem Speichel von Insekten, die Säugetierblut saugen, bevorzugt aus *Triatoma pallidipennis,* isolierbar ist,
a) wobei das Protein als aktives Protein eine N-terminale Sequenz wie folgt besitzt: 
Ala • Glu • Gly • Asp • Asp • Cys • Ser • Leu • Glu • Lys • Ala • Met •
5 10
Gly • Asp • Phe • Lys • Pro • Glu • Glu • Phe • Phe • ....
15 20
 oder
b) wobei das Protein als aktives Protein allelische Modifikationen der zuvor unter a) genannten N-terminalen Aminosäure-Sequenz aufweist, wobei eine oder zwei Aminosäuren der N-terminalen Aminosäure-Sequenz substituiert, deletiert oder insertiert sind, ohne dabei die Aktivität des aktiven Proteins wesentlich zu beeinflussen,
oder
c) wobei das Protein als aktives Protein posttranslationale Modifikationen der N-terminalen Sequenzen unter a) und b) aufweist, die nicht wesentlich die Aktivität des aktiven Proteins beeinflussen.

### SEQUENZEN DER REIFEN PROTEINE

Die Erfindung umfaßt weiterhin ein Protein, das ein Thrombininhibitor ist und
d) das als aktives reifes Protein eine der folgenden Sequenzen besitzt:
   i) Sequence Identifer No. 1 (Sequenz Protokoll Nr. 1);
   ii) Sequence Identifer No. 2 (Sequenz Protokoll Nr. 2);
   iii) Sequence Identifer No. 3 (Sequenz Protokoll Nr. 3) und
   iv) Sequence Identifer No. 4 (Sequenz Protokoll Nr. 4);
   oder
e) das als aktives reifes Protein allelische Modifikationen einer der zuvor unter d) genannten Aminosäure-Sequenzen aufweist, wobei wenigstens eine Aminosäure der Aminosäure-Sequenz substituiert, deletiert oder insertiert ist, ohne dabei die Aktivität des aktiven Proteins wesentlich zu beeinflussen,
   oder
f) das als aktives reifes Protein posttranslationale Modifikationen einer der Sequenzen unter d) und e) aufweist, die nicht wesentlich die Aktivität des aktiven Proteins beeinflussen.

Alle allelischen Modifikationen, die die Substitutionen, die Deletionen und/oder die Insertionen von bis zu 30 Aminosäuren umfassen, gehören zur Gruppe der erfindungsgemäßen Proteine. Bevorzugt sind Deletionen, Substitutionen und/oder Insertionen von bis zu 20 Aminosäuren, mehr bevorzugt von bis zu 10 Aminosäuren, am meisten bevorzugt sind die Deletionen, Substitutionen und/oder Insertionen von einer, zwei, drei, vier, fünf, sechs, sieben, acht oder neun Aminosäuren.

### SEQUENZEN DER REIFEN PROTEINE MIT SIGNALSEQUENZ

Eine weitere Ausführungsform der erfindungsgemäßen Proteine bestehen in einem Protein, das aus einer Signalsequenz und einem reifen erfindungsgemäßen Protein besteht,
g) wobei das Protein eine der fongenden Sequenzen besitzt:
   i) Sequence Identifier No. 5 (Sequenz Protokoll Nr. 5);
   ii) Sequence Identifier No. 6 (Sequenz Protokoll Nr. 6);
   iii) Sequence Identifier No. 7 (Sequenz Protokoll Nr. 7) und
   iv) Sequence Identifier No. 8 (Sequenz Protokoll Nr. 8);
   oder
h) wobei das Protein allelische Modifikationen einer der zuvor unter g) genannten Aminosäure-Sequenzen aufweist, wobei wenigstens eine Aminosäure der Aminosäure-Sequenz substituiert, deletiert oder insertiert ist, ohne dabei die Aktivität des reifen aktiven Proteins wesentlich zu beeinflussen,
   oder
i) wobei das Protein posttranslationale Modifikationen einer der Sequenzen unter g) und h) aufweist, die nicht westenlich die Aktivität des aktiven reifen Proteins beeinflussen.

Alle allelischen Modifikationen, die die Substitutionen, die Deletionen und/oder die Insertionen von bis zu 32 Aminosäuren umfassen, gehören zur Gruppe der erfindungsgemäßen Proteine. Bevorzugt sind Deletionen, Substitutionen und/oder Insertionen von bis zu 21 Aminosäuren, mehr bevorzugt von bis zu 11 Aminosäuren, am meisten bevorzugt sind die Deletionen, Substitutionen und/oder Insertionen von einer, zwei, drei, vier, fünf, sechs, sieben, acht oder neun Aminosäuren.

Am meisten bevorzugt sind erfindungsgemäße Proteine, die rekombinante Proteine sind. Dabei können die Proteine glykosyliert sein.

Die erfindungsgemäßen Protein umfassen die reifen Proteine und die entsprechenden Vorläuferproteine, welche sich aus einer "Signal-Sequence" (Signal-Sequenz) und der Sequenz des reifen Proteins zusammensetzen. Dabei geht die "Signal-Sequenz" der Sequenz des reifen Proteins voraus. Das reife Protein beginnt mit der zuvor genannten N-terminalen Sequenz unter Punkt a). Die "Signal-Sequenz" ist für die Durchdringung des endoplasmatischen Retikulums erforderlich.

### cDNA ODER DNA KODIEREND FÜR DIE ERFINDUNGSGEMÄßEN PROTEINE

Die Erfindung umfaßt weiterhin auch cDNA oder DNA, die einen reifen Thrombininhibitor kodiert,
aa) wobei die cDNA oder DNA eine der folgenden Aminosäure-Sequenzen kodiert:
   i) Sequence Identifier No. 1 (Sequenz Protokoll Nr. 1);
   ii) Sequence Identifier No. 2 (Sequenz Protokoll Nr. 2);
   iii) Sequence Identifier No. 3 (Seqeunz Protokoll Nr. 3) und
   iv) Sequence Idnetifier No. 4 (Sequenz Protokoll Nr. 4),
   oder
bb) wobei die cDNA oder DNA allelische Modifikationen einer der Aminosäure-Sequenzen unter aa) kodiert,
   worin wenigstens eine Aminosäure der Aminosäure-Sequenz substituiert, deletiert oder insertiert ist, ohne dabei dei Aktivität des aktiven Proteins wesentlich zu beeinflussen.

Die allelischen Modifikationen sind zuvor unter dem Punkt "Sequenzen der reifen Proteine" definiert worden.

Weiterhin umfaßt die Erfindung eine cDNA oder DNA, die einen Thrombininhibitor kodiert,
cc) wobei die cDNA oder DNA eine der fogenden Nukleotidsequenzen besitzt:
   i) Sequence Identifier No. 9 (Sequenz Protokoll Nr. 9);
   ii) Sequence Identifier No. 10 (Sequenz Protokoll Nr. 10);
   iii) Sequence Identifier No. 11 (Seqeunz Protokoll Nr. 11) und
   iv) Sequence Idnetifier No. 12 (Sequenz Protokoll Nr. 12),
   oder
dd) wobei die cDNA oder DNA eine allelische Modifikation einer der Nukleotidsequenzen unter cc) aufweist, wobei wenigstens ein Nukleotid substituiert, deletiert oder insertiert ist, ohne dabei die Aktivität des Proteins, das von der allelischen Modifikation der Nukleotidsequenz unter cc) kodiert wird, wesentlich zu beeinflussen.

Alle DNA-Konstrukte zählen auch dann zu den aufgezählten erfindungsgemäßen Sequenzen, wenn solche Nukleotide ausgetauscht werden, die aufgrund des degenerierten Kodes dieselbe Aminosäure kodieren. Der Austausch derartiger Nukleotide ist offensichtlich und die sich entsprechenden Aminosäuren sind in jedem Biochemielehrbuch offenbart. (R. KNIPPERS, 1982, 3. Auflage, Molekulare Genetik, Georg Thieme Verlag)

Alle allelischen Modifikationen, die zu einer Veränderung der Aminosäure-Sequenz führen, gehören zur Erfindung, sofern diese Modifikationen die Substitutionen, die Deletionen und/oder die Insertionen von bis zu 30 Aminosäuren umfassen. Bevorzugt sind Deletionen, Substitutionen und/oder Insertionen von bis zu 20 Aminosäuren, mehr bevorzugt von bis zu 10 Aminosäuren, am meisten bevorzugt sind die Deletionen, Substitutionen und/oder Insertionen von einer, zwei, drei, vier, fünf, sechs, sieben, acht oder neun Aminosäuren.

Weiterhin umfaßt die Erfindung auch ein genetisches Material, das neben der das reife Protein kodierenden Sequenz ebenfalls auch eine Signalsequenz enthält. Diese Signalsequenz ist in der cDNA-Bank gefunden worden, auch andere Signalsequenzen sind denkbar, die eine Expression und Sekretion des Proteins erlauben.

Somit umfaßt die Erfindung eine cDNA oder DNA, die einen Thrombininhibitor mit Signalsequenz kodiert,
ee) wobei die cDNA oder DNA eine der folgenden Nukleotidsequenzen besitzt:
   i) Sequence Identifier No. 13 (Sequenz Protokoll Nr. 13);
   ii) Sequence Identifier No. 14 (Sequenz Protokoll Nr. 14);
   iii) Sequence Identifier No. 15 (Seqeunz Protokoll Nr. 15) und
   iv) Sequence Identifier No. 16 (Sequenz Protokoll Nr. 16),
   oder
ff) wobei die cDNA oder DNA eine allelische Modifikation einer der Nukleotidsequenzen unter ee) aufweist, wobei wenigstens ein Nukleotid substituiert, deletiert oder insertiert ist, ohne dabei dei Aktivität des reifen Proteins, das einschießlich seiner Signalsequenz von der allelischen Modifikation der Nukleotid-sequenz unter ee) kodiert wird, wesentlich zu beeinflussen.

Die allelische Modifikationen sind zuvor unter dd) definiert worden.

Wenn die Aktivität des Proteins angegeben wird, um festzustellen, ob die allelische Modifikation unter die Gruppe der erfindungsgemäßen Proteine zählt, so ist immer das reife Portein zu messen, auch wenn die Signalsequenz ebenfalls angeführt ist. Sollte die Siganlsequenz angegeben sein, so ist die Funktion immer an dem Protein zu messen, daß nach Entfernen der Signalsequenz erhalten wird.

Weiterhin umfaßt die Erfindung Bindungsmoleküle (zum Beispiel Peptide oder deren Derivate), Einzelketten-Proteine (Single Chain Proteins), Antikörper oder Fragmente der Antikörper, die Domänen auf dem reifen, erfindungsgemäßen Protein spezifisch erkennen. Wenn das gereinigte erfindungsgemäße Protein vorliegt, ist es für den Fachmann leicht möglich, monoklonale Antikörper herzustellen. Dabei wird die bekannte Methode von Köhler und Milstein und deren Weiterführungen angewendet. Dabei wird im einzelnen in konventioneller Methode eine Maus mit dem gereinigten Protein mehrfach immunisiert, die Milzzellen entnommen und mit geeigneten Tumorzellen fusioniert. Die Hybride werden anschließend selektioniert.

Die Proteine der Erfindung können aus dem Speichel der Raubwanze *Triatoma pallidipennis* isoliert werden. Die Reinigung erfolgt durch eine Gelfiltration und eine anschließende Affinitätschromatographie an Thrombin-Sepharose (siehe Beispiel 1). Die Proteine haben die zuvor beschriebene Aminosäure-Sequenzen. Sie haben ein Molekulargewicht von ca. 18000 ± 3000 Da (siehe Beispiel 6). Der isoelektrische Punkt liegt im Bereich von pH 4,5 bis 5,2, wenn die im Beispiel 8 beschriebene Methode angewendet wird.

Die Proteine der Erfindung hemmen die Wirkung von Thrombin bei der Blutgerinnung und bei der Aktivierung von Plättchen und im amidolytischen Test. Die Test-Systeme sind in den Beispielen 2, 3, 4 und 9 beschrieben. Die Proteine hemmen die Gerinnung in einer Konzentration von 8 nmol/l bei einer Thrombinkonzentration von 1,27 nmol/l. Sie hemmen die Thrombin-induzierte Plättchen-Aggregation zu 100 % in einer Konzentration von 15 ng/ml. Diese Konzentration entspricht bei einer eingesetzten Thrombinkonzentration von 0,06 lU/ml = 0,812 pmol/ml (IU = Internationale Einheiten) einem molaren Verhältnis von Thrombin zum erfindungsgemäßen Protein von etwa 1:1. Dagegen hemmen die Proteine der Erfindung die Aktivität von Thrombin im amidolytischen Test bei einem Verhältnis von Thrombin zum erfindungsgemäßen Protein von 1:87 nur zu etwa 50%. Die Proteine der Erfindung verlängern in einer Konzentration von 35 nmol/l die Thrombin-Zeit (1 IU/ml) um das 5-fache.

Die Proteine der Erfindung sind spezifisch für Thrombin. Andere Serinproteasen (z.B. Faktor Xa oder Trypsin) werden auch bei einem 40-fachen Überschuß nicht nachweislich gehemmt (s. Beispiel 7).

### VEKTOREN MIT DER ERFINDGSGEMÄßEN DNA

Ein weiterer Teil der Erfindung ist ein Vektor, der eine erfindungsgemäße cDNA oder DNA, weiterhin einen passenden Promotor und gegebenenfalls einen passenden Enhancer enthält. Ebenfalls kann auch noch eine "Signal-Sequenz" umfaßt sein. Vektoren sind ausführlich in den europäischen Publikationen EP 0 480 651, EP 0 462 632 und EP 0 173 177 beschrieben.

Eine weitere Ausführungsform der Erfindung besteht in einer eukaryontischen oder prokaryontischen Wirtszelle, die mit einem erfindungsgemäßen Vektor transformiert ist.

### ALLELISCHE MODIFIKATIONEN

Die meisten Deletionen, Insertionen und Substitutionen scheinen keine durchgreifende Änderung in der Charakteristik des Proteins der Erfindung zur Folge zu haben. Da es schwer ist, den genauen Effekt einer Substitution, einer Deletion oder einer Insertion im voraus anzugeben, muß die Funktion des veränderten Proteins mit der Funktion des erfindungsgemäßen Proteins verglichen werden. Die hierfür zu verwendenden Methoden sind beispielhaft in den Beispielen 2 bis 4 und 9 angegeben. Als Standard dient das Protein gemäß der Seq. Id. No. 1 bis 4, ebenfalls auch das Protein, das nach Beispiel 1 gereinigt wird, und auch die Reinigungsmethode des Beispiels 1 für das Vergleichsprotein.

Der genetische Code ist degeneriert, das bedeutet, daß die meisten Aminosäuren von mehr als einem Codon aus drei Nukleotiden kodiert werden. Daher führen einige allelische Modifikationen auf der Ebene der Nukleotide nicht zu einer Änderung der Aminosäure-Sequenz. Daher ereignen sich allelische Modifikationen vornehmlich auf der Ebene der DNA und können sich sekundär auf die Aminosäure-Sequenz auswirken.

Die cDNA- oder DNA-Sequenzen, die die erfindungsgemäßen Proteine kodieren, können gemäß konventioneller Techniken modifiziert werden, um Varianten der erfindungsgemäßen Proteine herzustellen, die im wesentlichen die gleiche Aktivität wie die beschriebenen und charakterisierten Proteine der Erfindung besitzen. Dabei wird die Aktivität so gemessen, wie es in den Beispielen 2 bis 4 und 9 beschrieben ist.

Aminosäuren können wie in der Tabelle 1 dargestellt substituiert werden, ohne dabei die Funktion des Proteins wesentlich zu beeinflussen. In jedem einzelnen Fall ist durch den Aktivitätstest zu entscheiden, welchen Einfluß die Veränderung auf die Funktion des Proteins hat.

**Tabelle 1**

| ÜBLICHE SUBSTITUIERUNG VON AMINOSÄUREN IN EINEM PROTEIN | |
|---|---|
| URSPRÜNGLICHE AMINOSÄURE | BEISPIELSWEISE VORGENOMMENE SUBSTITUIERUNG |
| Ala | Gly, Ser |
| Arg | Lys |
| Asn | Gln, His |
| Asp | Glu |
| Cys | Ser |
| Gln | Asn |
| Glu | Asp |
| Gly | Ala, Pro |
| His | Asn, Gln |
| Ile | Leu, Val |
| Leu | Ile, Val |
| Lys | Arg, Gln, Glu |
| Met | Leu, Tyr, Ile |
| Phe | Met, Leu, Tyr |
| Ser | Thr |
| Thr | Ser |
| Trp | Tyr |
| Tyr | Trp, Phe |
| Val | Ile, Leu |

Die Funktionen oder die immunologische Identität werden wesentlich verändert, wenn Substituenten gewählt werden, die bei der Substituierung weniger konservativ als die in Tabelle 1 gezeigten Aminosäuren sind. Derartige wesentlichen Veränderungen lassen sich durch Substituierungen mit Aminosäuren erzielen, die sich mehr in ihrer Struktur und in den funktionellen Gruppen unterscheiden. Wesentliche Veränderungen wirken sich dahingehend aus, daß die dreidimensionale Struktur verändert wird und/oder daß zum Beispiel die Faltblatt-Struktur oder die helikale Struktur beeinflußt wird. Auch Wechselwirkungen der Ladungen und der hydrophoben Ketten sind bei den Veränderungen zu beachten.

Die Mutationen werden durch die Homologie (Similarity) zweier zum Vergleich anstehender Proteine definiert. Der Ausdruck Homologie umfaßt ähnlicne Aminosäuren (zum Beispiel Tabelle 1) und Lücken in den Sequenzen der Aminosäuren (Homologie = similarity). Die erfindungsgemäßen Proteine haben Aminosäure-Sequenzen, die eine Homologie von wenigstens 80 %, bevorzugt 90 %, mehr bevorzugt 95 % und am meisten bevorzugt 98 % der erfindungsgemäßen Strukturen besitzt, wie sie durch die Sequenzen unter d) oder g) (Seq. Id. No. 1 bis 8) definiert sind und wie sie weiterhin nach der Reinigung gemäß Beispiel 1 erhalten werden.

Wie zuvor erwähnt, umfaßt die Erfindung auch Modifikationen der DNA oder cDNA. Diese modifizierten Sequenzen hybridisieren unter stringenten Bedingungen mit den DNA-Sequenzen, die die erfindungsgemäßen Proteine kodieren (siehe Sequenzen unter aa); cc) und ee)). Die cDNA- oder DNA-Sequenzen haben Nukleotid-Sequenzen, die eine Identität einschließlich kurzer (bis 15 Nukleotide) Deletionen und Insertionen von wenigstens 70 %, bevorzugt 82 %, mehr bevorzugt 90 % und am meisten bevorzugt von 95 % mit den erfindungsgemäßen cDNA- oder DNA-Sequenzen besitzen (siehe aa), cc) und ee)). Die Identität einschließlich der kurzen (bis 15 Nukleotide) Deletionen und Insertionen kann durch eine Hybridisierung gemessen werden, wie sie in R. KNIPPERS, Molekulare Genetik, 1982, dritte Auflage, Georg Thieme Verlag Stuttgart, New York beschrieben ist.

### POSTTRANSLATIONALE MODIFIKATIONEN

Unter den zuvor erwähnten posttranslationalen Modifikationen versteht man Veränderungen, die während oder nach der Translation auftreten. Hierzu zählen die Glykosylierung, die Ausbildung von Disulfid-Brücken, die chemische Modifikationen der Aminosäuren, so zum Beispiel die Sulfatierung, die im Zusammenhang mit dem Hirudin beschrieben ist. (J.W. FENTON (1989) "Thrombin Interactions with Hirudin", Seminars in Thrombosis and Hemostasis **15:** 265 - 268)

Die Glykosylierung ist eine wesentliche Funktion des endoplasmatischen Retikulums und/oder des Golgi-Apparates. Die Sequenz und die Verästelung der Oligosaccharide wird in dem endoplasmatischem Retikulum gebildet und in dem Golgi-Apparat verändert. Die Oligosaccharide können N-verknüpfte Oligosaccharide (Asparagin-verknüpfte) oder O-verknüpfte Oligosaccharide (Serin-, Threonin- oder Hydroxylysin-verknüpfte) sein. Die Form der Glykosylierung ist von dem produzierenden Zelltyp und von der Art abhängig, von der der entsprechende Zelltyp stammt. Das Ausmaß und die Art der Glykosylierung kann durch Substanzen beeinflußt werden, wie es in der europäischen Publikation EP 0 222 313 beschrieben ist. Die Variierung der Glykosylierung kann die Funktion des Proteins verändern.

Proteine bilden häufig kovalente Bindungen innerhalb der Ketten aus. Diese Disulfid-Brücken werden zwischen zwei Cysteinen hergestellt. Dabei wird das Protein spezifisch gefaltet. Die Disulfid-Brücken stabilisieren die dreidimensionale Struktur der Proteine.

Weiterhin können die Aminosäuren so verändert werden, wie es in der internationalen Publikation WO 91/10684 beschrieben ist. Ebenfalls kann das Protein sulfatiert sein. Diese Veränderung ist im Zusammenhang mit Hirudin beschrieben.

### ISOLIERUNG UND HERSTELLUNG DER ERFINDUNGSGEMÄßEN PROTEINE

Die Erfindung umfaßt weiterhin ein Verfahren zur Herstellung der erfindungsgemäßen Proteine mit den folgenden Schritten:
Kultivierung einer Wirtszelle, die mit einem Vektor transformiert ist,
welcher eine erfindungsgemäße cDNA oder DNA enthält,
und
Isolieren und Reinigen des oder der Proteine.

Die Proteine werden bevorzugt gemäß Beispiel 1 gereinigt. Jedoch sind auch andere Isolierungs- und Reinigungs-Methoden möglich:
Methods of Enzymology, Volume **182:** Guide to Protein Purification, ed. Murray P. DEUTSCHER, Academic Press, 1990;
Protein Purification Application - A Practical Approach, ed. E.L.V. HARRIS and S. ANGEL, IRL-Press 1990;
Protein Purification, Principles and Practice, Ropert SCOPES, Springer-Verlag 1982; and
Protein Purification, Principles, High Resolution Methods and Applications, ed. H.-C. JANSON and L. RYDEN, VCH publishers 1989.

Die Erfindung umfaßt ebenfalls ein Verfahren zur Reinigung erfindungsgemäßer Proteine, wobei die Proteine isoliert, über wenigstens eine Säule gereinigt und anschließend eingeengt werden. Bevorzugt sind Chromatographie-Säulen oder Adsorptionschromatographie-Säulen.

Die Erfindung umfaßt darüber hinaus ein Verfahren zur Reinigung von erfindungsgemäßen Proteinen, wobei das Verfahren aus folgenden Schritten besteht:
Auftragen des Speichels auf eine "Superose 12 HR-Säule" und Eluieren und
erneute Auftragung auf einer CH-aktivierten Sepharose-Säule, an der zuvor Thrombin gekoppelt wurde, und Eluieren.
Die Reinigung ist ausführlich im Beispiel 1 beschrieben.

### VERWENDUNG ALS ARZNEIMITTEL

Die Proteine gemäß der Erfindung besitzen pharmakologische Effekte und sind deshalb als pharmazeutische Wirkstoffe verwendbar. Die Erfindung umfaßt ebenfalls ein Arzneimittel, das eines der erfindungsgemäßen Proteine oder ein Gemisch davon enthält. Weiterhin gehört zur Erfindung eine pharmazeutische Zusammensetzung, die eines der erfindungsgemäßen Proteine oder ein Gemisch erfindungsgemäßer Proteine enthält, in Gegenwart von pharmazeutisch verträglichen und annehmbaren Verbindungen und Trägern. Ebenfalls umfaßt die Erfindung eine pharmazeutische Zusammensetzung, die eines der pharmazeutisch aktiven, erfindungsgemäßen Proteine oder deren Gemisch und ein pharmazeutisch verträgliches Salz oder einen pharmazeutisch verträglichen Träger enthält.

Insbesondere zeigen die erfindungsgemäßen Proteine gemäß der Sequenz-Protokolle Nr. 1 bis 4 eine Inhibierung der Thrombinaktivität.

Die Inhibierung der Thrombinaktivität kann im Gerinnungstest (siehe Beispiel 2), im Blutplättchen-Aggregationstest (siehe Beispiel 3), im amidolytischen Test (siehe Beispiel 4) und durch Messen der Thrombin-Zeit (siehe Beispiel 9) nachgewiesen werden. Die Messung der Thrombin-Zeit ist das bevorzugte Testsystem.

Die erfindungsgemäßen Proteine zeigen eine Verlängerung der Thrombinzeit bei Konzentrationen von 10 bis 60 nmol/l (Thrombinkonzentration 1 IU / ml). Bei einer Konzentration von 58 nmol/l erfolgt eine 9 - fache Verlängerung. Höhere Konzentrationen sind anwendbar, ohne das Testsystem zu stören. Somit sind die erfindungsgemäßen Proteine in Konzentrationen von 10 bis 200 nmol/l einsetzbar.

Die Versuchsergebnisse dieses *in vitro* Testes zeigen, daß die erfindungsgemäßen Proteine als Arzneimittel oder zur medizinischen Behandlung verwendet werden können. Diese Versuchsergebnisse lassen sich von dem *in vitro* Testsystem auf ein *in vivo* System übertragen, da es sich bei dem Gerinnungstest um eine etablierte Versuchsanordnung handelt. (M. TALBOT (1989) "Biology of Recombinant Hirudin, A New Prospect in the Treatment of Thrombosis" Seminars in Thrombosis and Hemostasis **15:** 293 - 301) Die Proteine der Erfindung können deshalb zur Behandlung und Prävention von Thrombosen, instabiler Angina oder Arteriosklerose, oder zur Prävention eines Wiederverschlusses von Gefäßen nach PTCA/PTA (Angioplastie mit einem Ballonkatheter) oder zur Verhinderung der Blutgerinnung bei der Hämodialyse dienen. Die Proteine der Erfindung können als ein antithrombotisches und/oder antiarteriosklerotisches Medikament bei Säugern, insbesondere Menschen, zur Behandlung von thrombotischen und/oder arteriosklerotischen Beschwerden eingesetzt werden. Diese können beim Reißen von arteriosklerotischen Aggregationen (Plaques), bei Zerstörung von Endothelgewebe, wie zum Beispiel bei Sepsis, bei Transplantaten oder bei instabiler Angina, auftreten. Sie können ebenfalls verwendet werden, um erneute Verschlüsse nach der Behandlung von myokardialen Infarkten und/oder bei Fibrinolyse oder Angioplastie zu vermeiden. Dabei kann das Protein der Erfindung vor, bei und/oder nach der Einführung des Katheters verabreicht werden.

Die Erfindung liefert weiterhin
(i) die Verwendung eines der erfindungsgemäßen Proteine oder deren Gemisch zur Herstellung eines Medikaments zur Behandlung von
   Thrombosen, instabiler Angina oder Arteriosklerose, oder zur Prävention eines Wiederverschlusses von Gefäßen nach PTCA/PTA oder zur Verhinderung der Blutgerinnung bei der Hämodialyse;
(ii) ein Verfahren zur Behandlung von
   Thrombosen, instabiler Angina oder Arteriosklerose, oder zur Prävention eines Wiederverschlusses von Gefäßen nach PTCA/PTA oder Thrombolyse oder zur Verhinderung der Blutgerinnung bei der Hämodialyse, welches Verfahren eine Verabreichung einer Proteinmenge gemäß der Erfindung umfaßt, wobei die Menge die Krankheit unterdrückt, und wobei die Proteinmenge einem Patienten gegeben wird, der ein solches Medikament benötigt;
(iii) eine pharmazeutische Zusammensetzung zur Behandlung von
   Thrombosen, instabiler Angina oder Arteriosklerose, oder zur Prävention eines Wiederverschlusses von Gefäßen nach PTCA/PTA oder Thrombolyse oder zur Verhinderung der Blutgerinnung bei der Hämodialyse, welche Behandlung eines der erfindungsgemäßen Proteine oder deren Gemisch und wenigstens einen pharmazeutisch verträglichen Träger und Zusatz umfaßt.

Für diese therapeutische Wirkung sind unterschiedliche Dosen geeignet. Sie hängen beispielsweise von dem verwendeten Protein, von dem Wirt, von der Art der Verabreichung und von der Art und der Schwere der zu behandelnden Zustände ab.
Im allgemeinen sind jedoch bei Tieren zufriedenstellende Resultate zu erwarten, wenn die tägliche Dosen einen Bereich von 2 µg bis 2000 µg pro kg Körpergewicht umfassen. Bei größeren Säugetieren, beispielsweise dem Menschen, liegt eine empfohlenen tägliche Dosis im Bereich von 2 bis 2000 µg pro kg Körpergewicht, wenn das nach Beispiel 1 gereinigte Protein verwendet wird. Zum Beispiel wird diese Dosis zweckmäßigerweise in Teildosen bis zu viermal täglich verabreicht. Die täglichen Dosen bei der kurzzeitigen Behandlung von akuten Thromben können mit 20 bis 2000 µg pro kg Körpergewicht höher als die Dosen bei der chronischen Behandlung mit 2 bis 200 µg pro kg Körpergewicht ausfallen.
Ebenfalls sind zufriedenstellende Resultate zu erwarten, wenn das Protein der Erfindung subkutan verabreicht wird. Bevorzugt ist die gezielte Injektion in den Teil des Körpers, in dem sich Thromben gebildet haben.

Die erfindungsgemäßen Proteine können auf jedem üblichen Weg verabreicht werden, insbesondere in Form von Injektionslösungen oder Suspensionen.

Die vorliegende Erfindung stellt pharmazeutische Zusammensetzungen zur Verfügung, die eines der erfindungsgemäßen Proteine oder deren Gemisch und wenigstens einen pharmazeutisch verträglichen Träger oder Zusatz umfassen. Solche Zusammensetzungen können nach bekannten Verfahren hergestellt werden. Dabei ist auf Remington's Pharmaceutical Science, 15th ed Mack Publishing Company, East Pennsylvania (1980) hinzuweisen.

Die Versuchsergebnisse werden anhand der Figuren verdeutlicht, die im einzelnen folgendes zeigen:
Figur 1: Verlängerung der Thrombin-Zeit bei Zugabe von erfindungsgemäßem Protein aus Triatoma pallidipennis.
Figur 2: Inhibierung der Thrombinaktivität im amidolytischen Test (Triatoma = Trombininhibitor aus Triatoma pallidipennis)
Figur 3: Inhibierung der Thrombinaktivität im Gerinnungstest.

### BEISPIELE

### Beispiel 1

### Gewinnung des Speichels von Triatoma pallidipennis und Reinigung des Proteins der Erfindung

Die Raubwanzen werden durch mechanische Stimulation ihrer Probszis zur Absonderung ihres Speichels angeregt. Der Speichel wird auf einer Glasplatte aufgefangen und mit einer silikonisierten, ausgezogenen Pasteurpipette gesammelt.

Der Speichel wird gefriergetrocknet und in einer Konzentration von 2,5 mg/ml in destilliertem Wasser gelöst. 2 ml dieser Lösung werden über eine "Superose 12 HR 16/50"-Säule (Pharmacia) in 10 mmol/l Tris/HCI, pH 7,4, 0,0001 % "Pluronic F68" gelfiltriert. Die im Gerinnungstest (siehe Beispiel 2) aktiven Fraktionen werden vereinigt und auf eine CH-aktivierte Sepharose (Pharmacia), an die zuvor Thrombin nach den Angaben des Herstellers gekoppelt wurde, aufgetragen. Das Protein der Erfindung bindet an diese mit Thrombin versehenen Säule. Zunächst wird die Säule mit Tyrode-Puffer (ohne Serumalbumin) gewaschen, dann wird erst mit 10 mmol/l Na-Acetat, pH 4,5, und dann mit 10 mmol/l GLycin, pH 2,5, eluiert. In den Eluaten wird der pH-Wert auf 7 eingestellt. Das 10 mmol/l Glycin Eluat enthält das gereinigte Protein der Erfindung. Es ist aktiv im Gerinnungstest (siehe Beispiel 2), im Plättchen-Aggregationstest (siehe Beispiel 3), im amidolytischen Test (siehe Beispiel 4) und verlängert die Thrombin-Zeit (siehe Beispiel 9). Die Präparation enthält keine in der SDS-Gelelektrophorese detektierbare Verunreinigungen.

### Beispiel 2

### Inhibierung der Thrombinaktivität im Gerinnungstest

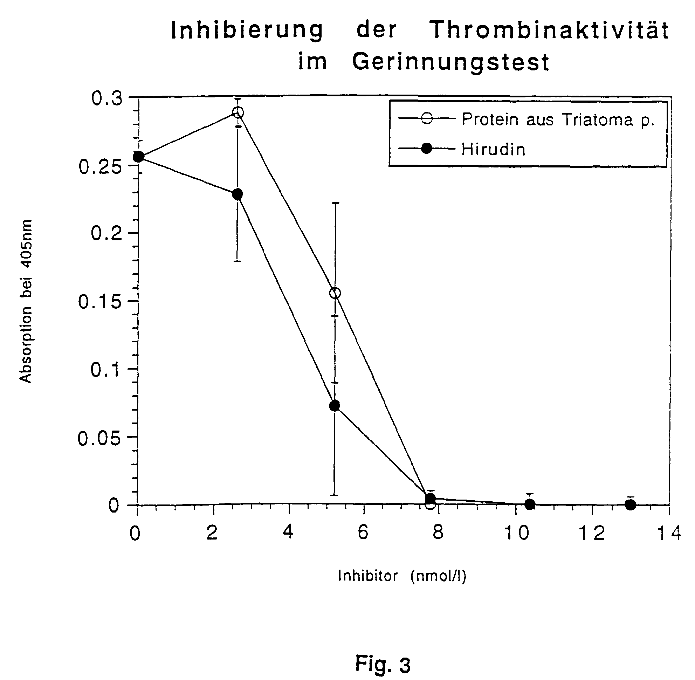

In eine Mikrotiterplatte, die mit Rinderserumalbumin beschichtet ist (0,1 % in 0,1 mol/l NaHCO₃, pH 9,5), werden 80µl 20 mmol/l HEPES, pH 7,4; 0,15 mmol/l NaCl; 20 µl 20 mmol/l CaCl₂; 100 µl einer verdünnten Lösung des Proteins der Erfindung (5-50 ng) und 20 µl Thrombin (0,03 IU = 0,03 Internationale Einheiten) pipettiert. Nach einer Inkubation 2 Minuten bei 37 °C werden 100 µl Fibrinogen (5 mg/ml) zugegeben und 40 Minuten bei 37 °C inkubiert. Anschließend wird die Absorption bei 405 nm gemessen. 45 ng ( =8 nmol/l) des gereinigten Proteins der Erfindung hemmen die Fibrinogenspaltung vollständig. Unter den gleichen Testbedingungen zeigt Hirudin die gleiche Wirksamkeit (vollständige Hemmung mit 8 nmol/l). (siehe Figur 3)

### Beispiel 3

### Inhibierung der Thrombinaktivität im Blutplättchen-Aggregationstest

500 µl gelfiltrierte Blutplättchen (300000/ml) werden mit dem Protein dieser Erfindung (5-100 ng/ml) für 1 Minute bei 37 °C inkubiert. Dann wird die Aggregation mit Thrombin (0,06 lU/ml) induziert und die Aggregation in einem Aggregometer aufgezeichnet. Messungen mit dem gereinigten Protein der Erfindung zeigen, daß bei einer Konzentration von 15 ng/ml die Aggregation zu 100 % gehemmt wird.

### Beispiel 4

### Inhibierung der Thrombinaktivität im amidolytischen Test

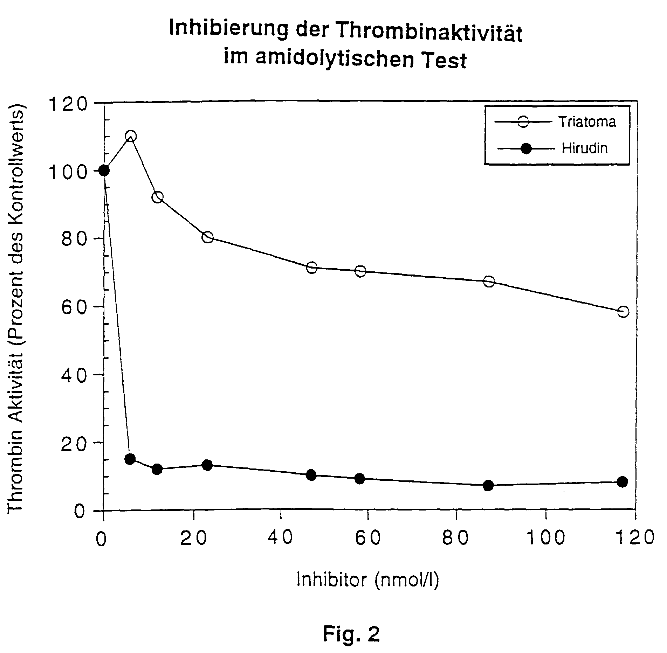

In einer Mikrotiterplatte werden 80 µl 100 mmol/l Tris/HCL, pH 7,4; 150 mmol/l NaCl und 0,03 IU (1,35 nmol/l) Thrombin und 100 µl einer verdünnten Lösung des Proteins der Erfindung (32 - 630 ng) 10 Minuten bei 37 °C inkubiert und dann mit 100 µl (50 nmol) des Substrates S2238 (Kabi Vitrum) versetzt. Nach einer Inkubation von 30 Minuten bei 37 °C wird die Absorption bei 405 nm gemessen. 630 ng (117 nmol/l) hemmen die Aktivität des Thrombins zu ungefähr 50 %. Hirudin hemmt in einer Konzentration von 6 nmol/l die Aktivität des Thrombins zu 85 %. (siehe Figur 2)

### Beispiel 5

### Bestimmung der N-terminalen Aminosäure-Sequenz

Das gereinigte Protein der Erfindung wurde in einem automatischen Aminosäuresequenator (Applied Biosystems, Inc.) nach den Instruktionen des Herstellers sequenziert. Die Sequenz der Aminosäuren 1 bis 21 (vom N-Terminus) ist: Ala-Glu-Gly-Asp-Asp-Cys-Ser-Leu-Glu-Lys-Ala-Met-Gly-Asp-Phe-?-Pro-Glu-Glu-Phe-Phe. "?" bedeutet, nicht mit vollständiger Sicherheit identifizierbar. Bei dieser Aminosäure handelt es sich mit einiger Wahrscheinlichkeit um Lysin.

### Beispiel 6

### SDS-Gelelektrophorese und Bestimmung des Molekulargewichts

Das Protein der Erfindung wurde im reduziertem (Reduktion mit Dithiothreit) und im nicht reduziertem Zustand zusammen mit den Molekulargewichtsmarkern (Electrophoresis Calibration Kit von Pharmacia) Phosporylase b (94 kDa), Albumin (67 kDa), Ovalbumin (43 kDa), Carbonic Anhydrase (30 kDa), Trypsin Inhibitor (20,1 kDa) und Lactalbumin (14,4 kDa) auf ein 12,5 %-iges SDS-Poly-acrylamidgel aufgetragen und nach der Elektrophorese nach Laemmli (1970, Nature 227, 680-685) mit Coomassie brillant blue gefärbt. Im reduzierten Zustand wandert das Protein der Erfindung während der Elektrophorese nur geringfügig oberhalb des Trypsin Inhibitors. Das entspricht einem Molekulargewicht von etwa 21000 Da. Im nicht reduziertem Zustand wandert das Protein der Erfindung zwischen dem Trypsin Inhibitor und Lactalbumin, was einem Molekulargewicht von etwa 18000 Da entspricht.

### Beispiel 7

### Das Protein der Erfindung hemmt nicht die Serinproteasen Faktor Xa und Trypsin.

Die Aktivität von Faktor Xa und von Trypsin werden mit dem folgenden Test gemessen. Zu 80 µl 50 mmol/l Tris/HCI pH 8,0, 227 mmol/l NaCl, werden für die Faktor Xa Bestimmung 0,004 IU (0,653 pmol) Faktor Xa (American Diagnostica) und 0,5 µg (28 pmol) des Proteins der Erfindung und für die Trypsin-Bestimmung 0,004 IU (0,019 pmol) Trypsin (Sigma) und 0,016 µg (0,817 pmol) des Proteins der Erfindung gegeben und für 2 Minuten bei 37 °C inkubiert. Nach Zugabe von 0,05 µmol des Substrates S2222 (Kabi Vitrum) wird für 20 Minuten bei 37 °C inkubiert und anschließend die Absorption bei 405 nm gemessen. Die Absorption dieser Ansätze ist genauso hoch wie die von Ansätzen ohne das Protein der Erfindung, d.h. das Protein hemmt weder die Aktivität von Faktor Xa noch die von Trypsin.

### Beispiel 8

### Isoelektrische Fokussierung

Das Protein der Erfindung wird auf ein Gel für isoelektrische Fokussierung im Bereich von pH 3 bis 9 (Pharmacia) aufgetragen und zusammen mit Standardproteinen (Calibration Kit pH 3 bis 10 von Pharmacia) fokussiert. Die Fokussierungsstelle des Proteins der Erfindung lag zwischen der des Sojabohnen-Trypsininhibitors (I.P. = 4,55) und der von β-Lactoglobulin A (I.P. = 5,2).

### Beispiel 9

### Verlängerung der Thrombin-Zeit

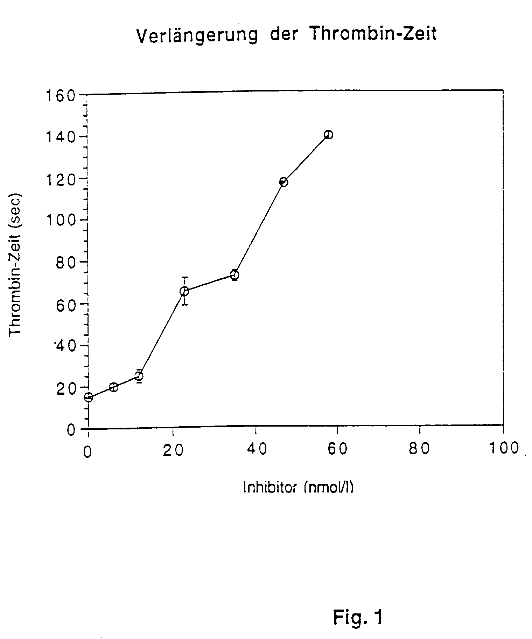

Die Thrombin-Zeit mißt die Aktivität von exogenem Thrombin, das zum Testplasma zugesetzt wird. Zu 0,1 ml Plasma werden 50 µl Lösung des Proteins der Erfindung in verschiedenen Verdünnungen (6 bis 58 nmol/l) und 50 µl Diäthylbarbiturat-Acetat-Puffer mit pH 7,6 gegeben und 1 Minute bei 37 °C inkubiert. Nach Zugabe von 0,1 ml Thrombin-Lösung (3 IU/ml) wird die Zeit bis zum Gerinnungseintritt gemessen (Biomatic 2000 Coagulometer von Sarstedt). Das Protein der Erfindung, das in einer Konzentration von 35 nmol/l vorliegt, verlängert die Gerinnungszeit im Vergleich zu einem Kontrollansatz um das 5-fache. (siehe Fig. 1)

### Beispiel 10

### Bestimmung von internen Aminosäuresequenzen nach Spaltung mit Lys C

Das gereinigte Protein der Erfindung (59 µg) wird mit 10% 2-Mercaptoethanol reduziert (2 Stunden bei Raumtemperatur unter N₂) und dann mit 4-Vinylpyridin umgesetzt (2 Stunden bei Raumtemperatur unter N₂). Nach einer Dialyse gegen 25 mmol/l Tris/HCI, pH 8,5; 1 mmol/l EDTA wird die Probe mit 1 µg Lys C (Boehringer Mannheim) versetzt und 6 Stunden bei 37 °C inkubiert. Dieser Spaltansatz wird auf eine Supersper RP-18, 4 µm (250 x 4 mm, MZ-Analysentechnik, Mainz)-Säule aufgetragen und mit einem Gradienten 0,1 % TFA in H₂O - 0,08 % TFA in 70 % Acetonitril eluiert (HPLC-Anlage von Waters). Die Absorption bei 280 nm und bei 214 nm wird aufgezeichnet und das Eluat wird fraktioniert. Die Fraktionen, die den Absorptions-Peaks entsprechen, werden zur Trockne gebracht und in 30 µl H₂O aufgenommen. Einzelne Fraktionen werden in einem automatischen Aminosäuresequenator (Applied Biosystems Inc.) nach den Instruktionen des Herstellers sequenziert. Dabei ergeben sich folgende Sequenzen (Start jeweils vom N-Terminus, "?" bedeutet, nicht eindeutig identifiziert): 
1.	Ala - Met - Gly - Asp - Phe - Lys - Pro - Glu - Glu - Phe - Phe - ? - Gly -
Thr - Arg(?) - Tyr - Leu - Ala
 (Die Bestimmung der Aminosäure Arg ist mit Unsicherheit behaftet.)

2.	Gly - Phe - Thr - Gln - Ile - Val - Glu - Ile - Gly - Tyr - Asn - Lys -

3.     Asn - Gly - Glu - Gln - Tyr - Ser - Phe  Lys -

### Beispiel 11

### Molekulare Klonierung von den erfindungsgemäßen cDNAs

Wenn die Sequenz des N-terminalen Endes des erfindungsgemäßen Proteins bekannt ist, läßt sich eine korrespondierende Nukleotidsequenz ermitteln. (siehe WO 90/07861)

### aa) Herstellung von spezifischen Proben bei der PCR

Um die erfindungsgemäßen cDNA zu ermitteln, wird zuerst der Primer (Vorlage, Startstück) synthetisiert, der von der zuvor im Edman-Abbau ermittelte Aminosäuresequenz abgeleitet wird. Die synthetisierten Oligonucleotidesequenzen des Primers werden genutzt, um mit der PCR (Polymerase Chain Reaction = Polymerase-Kettenreaktion) ein Fragment der erfindungsgemäßen cDNA zu amplifizieren. (siehe US-Patent 4,800,159)
Zwei Oligonukleotid-Primer werden hergestellt, indem die Nukleotid-Sequenz von der zuvor teilweise bestimmten Aminosäure-Sequenz des N-terminalen Bereichs des vollständigen erfindungsgemäßen Proteins und eines seiner Fragmente hergeleitet wird. Dabei werden die folgenden Aminosäure-Sequenzen verwendet:
N-terminale Sequenz des vollständigen Proteins: 
Ala - Glu - Gly - Asp - Asp - Cys - Ser - Leu - Glu - Lys -
                        5                                  10

Ala - Met - Gly - Asp - Phe
                        15
 und N-terminale Sequenz des Fragmentes:

Gly - Phe - Thr - Gln - Ile - Val - Glu - Ile - Gly - Tyr -
                        5                                  10

Asn - Lys
      12

Die Matritze (template) besteht aus der cDNA, die von einer poly-A⁺-RNA stammt, welche zuvor aus den Speicheldrüsen von *Triatoma pallidipennis* isoliert worden ist. (SAMBROCK et al.: Molekular Cloining (Chapter 7, pp 18 - 22, Cold Spring Harbor Laboratory Press, 1989)

Matritzen-Startstück (Sense primer):

5′-GCI GA(A/G) GGI GA(C/T) GA(C/T) TG(C/T) TCI CTI GA(A/G)
AA(A/G) GCI ATG GGI GA(C/T) TT-3′

Nicht-Matritzen- Startstück (Antisense primer): 
5′-TT (G/A)TT (G/A)TA ICC (G/A)AT (T/C)TC IAC (G/A)AT (T/C)TG
IGT (G/A)AA-3
A = Desoxyadenosin; C = Desoxycytidin;
G = Desoxyguanosin; T = Desoxythymidin und
I = Desoxyinosin

Die PCR setzt sich aus 40 Zyklen zusammen. Ein Zyklus sieht wie folgt aus:
a) 2 Minuten Denaturierung bei 94 °C,
b) 90 Sekunden Hybridisierung bei 52 °C und
c) 2 Minuten Verlängerung bei 72 °C.

Das durch das zuvor beschriebene Verfahren gewonnen PCR-Vermehrungprodukt weist auf einem Agarose-Gel eine Bande auf. Diese wird von dem Agarose-Gel, das einen niedrigen Schmelzpunkt aufweist, isoliert und direkt in das PCR Plasmid (Stratagene) überführt und subkloniert. Das Verfahren entspricht der Beschreibung aus den Protokoll des Herstellers (pCR-Script^{TM} SK(+) Cloning Kit von Stratagen). Die DNA-Sequenz des Insertionsproduktes entspricht der Sequenz des PCR-Produktes und wird so ermittelt, wie es in SANGER et al. Proc Natl Acad Sci USA (1977) **74:** 5463 - 5467 beschreiben ist.

### bb) Selektionsverfahren der cDNA-Bank und Isolierung des erfindungsgemäßen cDNA-Klons.

Etwa 400.000 primäre Klone aus der Speicheidrüsen-cDNA-Bank von *T. pallidipennis* (cDNA library) werden auf Nylonmembranen (Pall Biosupport East Hills, NY, USA) transferiert und gescreent, wie es der Hersteller vorschreibt. Eine markierte Probe, die mit Hilfe der zuvor beschriebenen PCR-Vermehrungsmethode gewonnen worden ist, wird dabei verwendet. Das Selektionsverfahren wird zweimal durchgeführt, gefolgt von eine Umwandlung der Phagen-DNA in Plasmid-DNA und eine Sequenzanalyse des Inserts (eingebaute DNA) dieses Plasmids. Dadurch werden vier cDNA-Sequenzen ermittelt, die in den Sequenz Identifier 13 bis 16 abgebildet sind, wobei lediglich die Triplika für die Aminosäuren -17 bis -9 bei Ti 28 und Ti 45 und das Triplika für die Aminosäure -18 bei Ti 12 fehlen.

Je beide Stränge der isolierten DNA werden sequenziert. Die von der kodierenden DNA abgeleitete Aminosäure-Sequenz stimmt vollständig mit der Aminosäure-Sequenz überein, die zuvor mittels des Edman-Abbaus von dem erfindungsgemäßen gereinigten Protein herausgefunden worden ist.

Der längste cDNA Klone (Ti12) beginnt mit den Nukleotiden TG, die einem unvollständigen Startkodon für Methionin entsprechen. Dieses wird offensichtlich, wenn ein weiterer isolierter Klon (Ti5) berücksichtigt wird, der bei einem Selektionierungsverfahren mit 600.000 Phagen derselben cDNA-Bank gefunden wurde, welche bei der Selektionierung von Ti12, Ti28 und Ti45 verwendet wurde.

**Tabelle 2**

| **Positionen, in denen die erfindungsgemäßen Proteine Unterschiede besitzten.** | | | | | |
|---|---|---|---|---|---|
| | | **Klon** | | | |
| | | **Ti5** | **Ti12** | **Ti28** | **Ti45** |
| Position | **8** | Ile | Leu | Leu | Leu |
| Position | **32** | Gly | Gly | Asp | Asp |
| Position | **72** | Val | Val | Val | Ala |
| Position | **74** | Asn | Asn | Asn | Lys |
| Position | **77** | Gly | Asp | Asp | Asp |
| Position | **86** | Ser | Gly | Gly | Gly |
| Position | **114** | Thr | Ile | Ile | Thr |
| Position | **127** | Leu | Phe | Phe | Phe |
| Position | **139** | Lys | Asn | Asn | Asn |

Die vier cDNA-Sequenzen des erfindungsgemäßen Proteins werden gemäß der zuvor beschreibenen Methode selektioniert, identifiziert und sequenziert. Lediglich 9 Positionen weisen Unterschiede in der abgeleiteten Aminosäure-Sequenz auf. Die Unterschiede sind in der Tabelle 2 aufgelistet.

Die Identität (Identity) und Ähnlichkeit (Similarity) der vier reifen erfindungsgemäßen Proteine ist etwa 95 oder 98%, je nachdem welcher Vergleichspartner gewählt wird.

### Beispiel 12

### Herstellung eines das erfindungsgemäße Protein kodierenden Plasmids und Expression und Reinigung des in E. coli hergestellten Proteins.

Sind die DNA-Sequenzen bekannt, so lassen sich passende Promotoren und gegebenenfalls passende Enhancer mit den jeweiligen DNA-Sequenzen verbinden, so daß dann ein brauchbarer Vektor vorliegt. (M. WIRTH, L. SCHUMACHER and H. HAUSER, in H.S. CONRADT (ed) Protein Glycosylation, Cellular Biotechnical and Analytical Aspects Vol 15, 49 - 52, VCH publishers, Weinheim, 1991); ebenfalls auch J. KRÄTZSCHMAR et al. (1992) Gene **116:** 281 - 284. Die Expression eines solchen Vektors ist in Eukaryonten (zum Beispiel Baby Hamster Kidney Cells) möglich. Weiterhin lassen sich die DNA-Sequenzen in passende prokaryontische Vektoren zur Expression in *E. coli* Stämmen einbauen.

### a) Herstellung des Plasmids

### (i) Vorüberlegung

Das Konstrukt zur Expression des erfindungsgemäßen rekombinanten Proteins in Prokaryonten wird hergestellt, indem das kommerziell erhältliche Plasmid pKK233-2 eingesetzt wird, das den IPTG-induzierbaren *trc* Promotor enthält. Der zur Expression geeignete Vektor umfaßt dieses Plasmid pKK233-2, in dem sowohl die kodierende Sequenz des erfindungsgemäßen Proteins als auch eine Signalsequenz inseriert sind. Die Signalsequenz ist eine modifizierte Signal-sequenz, die auf das sekretierte *E. coli* Protein Cyclophilin a zurückzuführen ist. Die Singalsequenz und die kodierende DNA wird stromabwärts (downstream) von dem Promotor eingesetzt, wodurch das pKK/cph entsteht (T. HAYANO (1991) Biochemistry **30:** 3041 - 3048).

Die kodierenden Sequenzen des erfindungsgemäßen Proteins werden in das Expressions-Plasmid inseriert und das so erhaltene Konstrukt (pKK/cph-Protein) wird für die Transformation von kompetenten *E. coli* JM 105 - Zellen benutzt.

### (ii) Konstruktion des Plasmids pKK/cph

Das folgende Paar von sich teilweise überlappenden und komplementären Oligodesoxynukleotiden wird verwendet, um die kodierende DNA für die Signal-sequenz von Cyclophilin a nachzubilden. Dabei wird das entsprechende C-terminale Ende der Cyclophilin-Sequenz modifiziert, um eine optimale Spaltstelle für die bakterielle Signalpeptidase zu bilden. Die Modifikation besteht darin, daß die letzten sieben Triplika des C-terminalen Endes gegen Triplika ausgetauscht werden, die folgende Aminosäure-Sequenz kodieren: Phe-Ser-Ala-Ser-Ala-Leu-Ala (R.E. DALBEY and G. von HEIJNE (1992) TIBS **17:** 474-478).

Sinn-tragende Oligonucleotid-Sequenz: (Sense oligo)

5′-GCGATAACAT GTTCAAAAGC ACCCTGGCGG CGATGGCTGC
TGTTTTCGCT CTGTCTG-3′;

Nicht-sinntragende Oligonukleotid-Sequenz: (Antisens oligo)

5′-CGCTATAAGC TTCTGCAGGC TAGCGCGCTC GCGCTGAAAG
CAGACACAGT CGAAACAG-3′

Nach dem Anlagern der Startersequenz und der anschließenden Komplettierung der Tochterstücke unter Verwendung von Taq Polymerase werden die DNA Fragmente mit *Af*/III und *Hin*dlll gespalten. Die Spaltungsstellen sind unterstrichen. Danach wird das DNA-Fragment zwischen der *Nco*l- und *Hin*dIII-Stelle des Plasmids pKK233-2 subkloniert. Die *Nhe*l-Stelle, die zusammen mit der *Hin*dIII Stelle die weitere Subklonierung von cDNA-Stücken erleichtert, wird durch Fettdruck kenntlich gemacht.

### (ii) Konstruktion des Plasmids mit dem erfindungsgemäßen Protein

Das folgende Paar an Startsequenzen (Primer) wird verwendet, um die kodierende Sequenz für das reife erfindungsgemäße Protein (Ti28 = Seq. Id. No 2) zu vervielfältigen. Dabei werden die *Nhe*l und *Hin*dIII Schnittstellen, die unterstrichen sind, für die Insertion in das Plasmid pKK/cph benötigt.

Sinn-tragende Oligonucleotid-Sequenz: (Sense oligo)

5′-GCGATAGCTA GCAGCAGAAG GTGACGAC-3′;

Nicht-sinntragende Oligonukleotid-Sequenz: (Antisens oligo)

5′-GCGATAGGAT CCAAGCTTAC TAACAAATTT CATTAGCATC
AGG-3′

Zehn Zyklen bestehend aus 2 Minuten bei 94 °C, 2 Minuten bei 30 °C und 2,5 Minuten bei 72 °C werden für die PCR (Polymerase Chain Reaktion = Polymerase-Ketten-Reaktion) durchgeführt, wobei 2 µg Matrizenstrang (Template) eingesetzt werden. Die vervielfältigte DNA wird auf einem niedrig-schmelzenden Agarose-Gel isoliert, mit *Nhe*l und *Hin*dIII gespalten und in das Plasmid pKK/cph überführt, das zuvor mit denselben Restriktionsendonukleasen gespalten wurde. Das so erhalte Konstrukt wird mit Hilfe einer Totalsequenzierung der kodierenden DNA und der die kodierende DNA flankierenden DNA überprüft.

### (iii) Transformation von E. coli.

*E. coli* JM 105 - Zellen werden unter Anwendung des CaCl₂ - Verfahrens transformiert. Dabei wird 1 µg pKK/cph DNA, die das erfindungsgemäße Protein kodiert, verwendet.

### b) Reinigung und Charakterisierung des mit E. coli hergestellten erfindungsgemäßen Proteins

Eine Kultur der transformierten *E. coli -* Zellen wird mit IPTG (1 mmol/l) versetzt und bei 37 °C für sechs Stunden inkubiert. (IPTG = Isopropyl-β-D-Thiogalactosid) Die Zellen werden abzentrifugiert und einem osmotischen Schock (Saccharose- und anschließende H₂O-Behandlung) unterworfen. Die so gewonnene Periplasmafraktion inhibiert im Gerinnungstest (vergleiche Beispiel 2) die Aktivität von Thrombin. Die inhibitorische Aktivität wird durch lonenaustauscherchromatographie an DE-52 Zellulose und Thrombinaffinitätschromatographie gereinigt. (vergleiche Beispiel 1)
Die gereinigte Fraktion hat die gleiche inhibitorische Aktivität wie das Speichelprotein. Es zeigt identisches Verhalten in einer SDS-Polyacrylamidelektrophorese (vergleiche Beispiel 6). Weiterhin stimmt es in der N-terminalen Aminosäuresequenz mit dem reifen Speichelprotein überein.

SEQ ID NO: 1
ART DER SEQUENZ:      Aminosäure-Sequenz
SEQUENZLÄNGE:         142 Aminosäuren
ART DES MOLEKÜLS:     reifes Protein Ti 12
URSPRÜNGLICHE HERKUNFT:   Speicheldrüse von Triatoma pallidipennis
EIGENSCHAFTEN:        als reifes Protein:Thrombininhibitor

Ala Glu Gly Asp Asp Cys Ser Leu Glu Lys Ala Met Gly Asp Phe
                5                   10                  15

Lys Pro Glu Glu Phe Phe Asn Gly Thr Trp Tyr Leu Ala His Gly
                20                  25                  30

Pro Gly Val Thr Ser Pro Ala Val Cys Gln Lys Phe Thr Thr Ser
                35                  40                  45

Gly Ser Lys Gly Phe Thr Gln Ile Val Glu Ile Gly Tyr Asn Lys
                50                  55                  60

Phe Glu Ser Asn Val Lys Phe Gln Cys Asn Gln Val Asp Asn Lys
                65                  70                  75

Asn Asp Glu Gln Tyr Ser Phe Lys Cys Lys Gly Ser Asp Asn Thr
                80                  85                  90

Glu Phe Glu Ala Asp Phe Thr Phe Ile Ser Val Ser Tyr Asp Asn
                95                  100                 105

Phe Ala Leu Val Cys Arg Ser Ile Ile Phe Thr Ser Gln Pro Lys
                110                 115                 120

Glu Asp Asp Tyr Leu Val Phe Glu Arg Thr Lys Ser Asp Thr Asp
                125                 130                 135

Pro Asp Ala Asn Glu Ile Cys
                140

SEQ ID NO: 2
ART DER SEQUENZ:        Aminosäure-Sequenz
SEQUENZLÄNGE:           142 Aminosäuren
ART DES MOLEKÜLS:       reifes Protein Ti 28
URSPRÜNGLICHE HERKUNFT:    Speicheldrüse von Triatoma pallidipennis
EIGENSCHAFTEN:          als reifes Protein:Thrombininhibitor

Ala Glu Gly Asp Asp Cys Ser Leu Glu Lys Ala Met Gly Asp Phe
                5                   10                  15

Lys Pro Glu Glu Phe Phe Asn Gly Thr Trp Tyr Leu Ala His Gly
                20                  25                  30

Pro Asp Val Thr Ser Pro Ala Val Cys Gln Lys Phe Thr Thr Ser
                35                  40                  45

Gly Ser Lys Gly Phe Thr Gln Ile Val Glu Ile Gly Tyr Asn Lys
                50                  55                  60

Phe Glu Ser Asn Val Lys Phe Gln Cys Asn Gln Val Asp Asn Lys
                65                  70                  75

Asn Asp Glu Gln Tyr Ser Phe Lys Cys Lys Gly Ser Asp Asn Thr
                80                  85                  90

Glu Phe Glu Ala Asp Phe Thr Phe Ile Ser Val Ser Tyr Asp Asn
                95                  100                 105

Phe Ala Leu Val Cys Arg Ser Ile Ile Phe Thr Ser Gln Pro Lys
                110                 115                 120

Glu Asp Asp Tyr Leu Val Phe Glu Arg Thr Lys Ser Asp Thr Asp
                125                 130                 135

Pro Asp Ala Asn Glu Ile Cys
                140

SEQ ID NO: 3
ART DER SEQUENZ: Aminosäure-Sequenz
SEQUENZLÄNGE: 142 Aminosäuren
ART DES MOLEKÜLS: reifes Protein Ti 45
URSPRÜNGLICHE HERKUNFT: Speicheldrüse von Triatoma pallidipennis
EIGENSCHAFTEN: als reifes Protein:Thrombininhibitor

Ala Glu Gly Asp Asp Cys Ser Leu Glu Lys Ala Met Gly Asp Phe
                5                   10                  15

Lys Pro Glu Glu Phe Phe Asn Gly Thr Trp Tyr Leu Ala His Gly
                20                  25                  30

Pro Asp Val Thr Ser Pro Ala Val Cys Gln Lys Phe Thr Thr Ser
                35                  40                  45

Gly Ser Lys Gly Phe Thr Gln Ile Val Glu Ile Gly Tyr Asn Lys
                50                  55                  60

Phe Glu Ser Asn Val Lys Phe Gln Cys Asn Gln Ala Asp Lys Lys
                65                  70                  75

Asn Asp Glu Gln Tyr Ser Phe Lys Cys Lys Gly Ser Asp Asn Thr
                80                  85                  90

Glu Phe Glu Ala Asp Phe Thr Phe Ile Ser Val Ser Tyr Asp Asn
                95                  100                 105

Phe Ala Leu Val Cys Arg Ser Ile Thr Phe Thr Ser Gln Pro Lys
                110                 115                 120

Glu Asp Asp Tyr Leu Val Phe Glu Arg Thr Lys Ser Asp Thr Asp
                125                 130                 135

Pro Asp Ala Asn Glu Ile Cys
                140

SEQ ID NO: 4
ART DER SEQUENZ: Aminosäure-Sequenz
SEQUENZLÄNGE: 142 Aminosäuren
ART DES MOLEKÜLS: reifes Protein Ti 5
URSPRÜNGLICHE HERKUNFT: Speicheldrüse von Triatoma pallidipennis
EIGENSCHAFTEN: als reifes Protein:Thrombininhibitor

Ala Glu Gly Asp Asp Cys Ser Ile Glu Lys Ala Met Gly Asp Phe
                5                   10                  15

Lys Pro Glu Glu Phe Phe Asn Gly Thr Trp Tyr Leu Ala His Gly
                20                  25                  30

Pro Gly Val Thr Ser Pro Ala Val Cys Gln Lys Phe Thr Thr Ser
                35                  40                  45

Gly Ser Lys Gly Phe Thr Gln Ile Val Glu Ile Gly Tyr Asn Lys
                50                  55                  60

Phe Glu Ser Asn Val Lys Phe Gln Cys Asn Gln Val Asp Asn Lys
                65                  70                  75

Asn Gly Glu Gln Tyr Ser Phe Lys Cys Lys Ser Ser Asp Asn Thr
                80                  85                  90

Glu Phe Glu Ala Asp Phe Thr Phe Ile Ser Val Ser Tyr Asp Asn
                95                  100                 105

Phe Ala Leu Val Cys Arg Ser Ile Thr Phe Thr Ser Gln Pro Lys
                110                 115                 120

Glu Asp Asp Tyr Leu Val Leu Glu Arg Thr Lys Ser Asp Thr Asp
                125                 130                 135

Pro Asp Ala Lys Glu Ile Cys
                140

SEQ ID NO: 5
ART DER SEQUENZ: Aminosäure-Sequenz
SEQUENZLÄNGE: 160 Aminosäuren
ART DES MOLEKÜLS: reifes Protein mit Signalsequenz Ti12
URSPRÜNGLICHE HERKUNFT: Speicheldrüse von Triatoma pallidipennis
EIGENSCHAFTEN: als reifes Protein:Thrombininhibitor

Met Lys Thr
-18 -17

Ile Ile Ala Val Thr Ile Phe Gly Ile Leu Thr Cys Ala Tyr Ala
-15 -10 -5 -1

Ala Glu Gly Asp Asp Cys Ser Leu Glu Lys Ala Met Gly Asp Phe
                5                   10                  15

Lys Pro Glu Glu Phe Phe Asn Gly Thr Trp Tyr Leu Ala His Gly
                20                  25                  30

Pro Gly Val Thr Ser Pro Ala Val Cys Gln Lys Phe Thr Thr Ser
                35                  40                  45

Gly Ser Lys Gly Phe Thr Gln Ile Val Glu Ile Gly Tyr Asn Lys
                50                  55                  60

Phe Glu Ser Asn Val Lys Phe Gln Cys Asn Gln Val Asp Asn Lys
                65                  70                  75

Asn Asp Glu Gln Tyr Ser Phe Lys Cys Lys Gly Ser Asp Asn Thr
                80                  85                  90

Glu Phe Glu Ala Asp Phe Thr Phe Ile Ser Val Ser Tyr Asp Asn
                95                  100                 105

Phe Ala Leu Val Cys Arg Ser Ile Ile Phe Thr Ser Gln Pro Lys
                110                 115                 120

Glu Asp Asp Tyr Leu Val Phe Glu Arg Thr Ile Lys Ser Asp Thr
                125                 130                 135

Asp Pro Asp Ala Asn Glu Ile Cys
                140

SEQ ID NO: 6
ART DER SEQUENZ: Aminosäure-Sequenz
SEQUENZLÄNGE: 160 Aminosäuren
ART DES MOLEKÜLS: reifes Protein mit Signalsequenz Ti 28
URSPRÜNGLICHE HERKUNFT: Speicheldrüse von Triatoma pallidipennis
EIGENSCHAFTEN: als reifes Protein:Thrombininhibitor

Met Lys Thr
-18

Ile Ile Ala Val Thr Ile Phe Gly Ile Leu Thr Cys Ala Tyr Ala
-15 -10 -9 -8 -5 -1

Ala Glu Gly Asp Asp Cys Ser Leu Glu Lys Ala Met Gly Asp Phe
                5                   10                  15

Lys Pro Glu Glu Phe Phe Asn Gly Thr Trp Tyr Leu Ala His Gly
                20                  25                  30

Pro Asp Val Thr Ser Pro Ala Val Cys Gln Lys Phe Thr Thr Ser
                35                  40                  45

Gly Ser Lys Gly Phe Thr Gln Ile Val Glu Ile Gly Tyr Asn Lys
                50                  55                  60

Phe Glu Ser Asn Val Lys Phe Gln Cys Asn Gln Val Asp Asn Lys
                65                  70                  75

Asn Asp Glu Gln Tyr Ser Phe Lys Cys Lys Gly Ser Asp Asn Thr
                80                  85                  90

Glu Phe Glu Ala Asp Phe Thr Phe Ile Ser Val Ser Tyr Asp Asn
                95                  100                 105

Phe Ala Leu Val Cys Arg Ser Ile Ile Phe Thr Ser Gln Pro Lys
                110                 115                 120

Glu Asp Asp Tyr Leu Val Phe Glu Arg Thr Lys Ser Asp Thr Asp
                125                 130                 135

Pro Asp Ala Asn Glu Ile Cys
                140

SEQ ID NO: 7
ART DER SEQUENZ: Aminosäure-Sequenz
SEQUENZLÄNGE: 160 Aminosäuren
ART DES MOLEKÜLS: reifes Protein mit Signalsequenz Ti 45
URSPRÜNGLICHE HERKUNFT: Speicheldrüse von Triatoma pallidipennis
EIGENSCHAFTEN: als reifes Protein:Thrombininhibitor

Met Lys Thr
-18

Ile Ile Ala Val Thr Ile Phe Gly Ile Leu Thr Cys Ala Tyr Ala
-15 -10 -9 -8 -5 -1

Ala Glu Gly Asp Asp Cys Ser Ile Glu Lys Ala Met Gly Asp Phe
                5                   10                  15

Lys Pro Glu Glu Phe Phe Asn Gly Thr Trp Tyr Leu Ala His Gly
                20                  25                  30

Pro Asp Val Thr Ser Pro Ala Val Cys Gln Lys Phe Thr Thr Ser
                35                  40                  45

Gly Ser Lys Gly Phe Thr Gln Ile Val Glu Ile Gly Tyr Asn Lys
                50                  55                  60

Phe Glu Ser Asn Val Lys Phe Gln Cys Asn Gln Ala Asp Lys Lys
                65                  70                  75

Asn Asp Glu Gln Tyr Ser Phe Lys Cys Lys Gly Ser Asp Asn Thr
                80                  85                  90

Glu Phe Glu Ala Asp Phe Thr Phe Ile Ser Val Ser Tyr Asp Asn
                95                  100                 105

Phe Ala Leu Val Cys Arg Ser Ile Thr Phe Thr Ser Gln Pro Lys
                110                 115                 120

Glu Asp Asp Tyr Leu Val Phe Glu Arg Thr Lys Ser Asp Thr Asp
                125                 130                 135

Pro Asp Ala Asn Glu Ile Cys
                140

SEQ ID NO: 8
ART DER SEQUENZ:     Aminosäure-Sequenz
SEQUENZLÄNGE:     160 Aminosäuren
ART DES MOLEKÜLS:     reifes Protein mit Signalsequenz Ti 5
URSPRÜNGLICHE HERKUNFT:     Speicheldrüse von Triatoma pallidipennis
EIGENSCHAFTEN:     als reifes Protein:Thrombininhibitor

Met Lys Thr
-18

Ile Ile Ala Val Thr Ile Phe Gly Ile Leu Thr Cys Ala Tyr Ala
-15                 -10                 -5                  -1

Ala Glu Gly Asp Asp Cys Ser Ile Glu Lys Ala Met Gly Asp Phe
                5                   10                  15

Lys Pro Glu Glu Phe Phe Asn Gly Thr Trp Tyr Leu Ala His Gly
                20                  25                  30

Pro Gly Val Thr Ser Pro Ala Val Cys Gln Lys Phe Thr Thr Ser
                35                  40                  45

Gly Ser Lys Gly Phe Thr Gln Ile Val Glu Ile Gly Tyr Asn Lys
                50                  55                  60

Phe Glu Ser Asn Val Lys Phe Gln Cys Asn Gln Val Asp Asn Lys
                65                  70                  75

Asn Gly Glu Gln Tyr Ser Phe Lys Cys Lys Ser Ser Asp Asn Thr
                80                  85                  90

Glu Phe Glu Ala Asp Phe Thr Phe Ile Ser Val Ser Tyr Asp Asn
                95                  100                 105

Phe Ala Leu Val Cys Arg Ser Ile Thr Phe Thr Ser Gln Pro Lys
                110                 115                 120

Glu Asp Asp Tyr Leu Val Leu Glu Arg Thr Lys Ser Asp Thr Asp
                125                 130                 135

Pro Asp Ala Lys Glu Ile Cys
                140

SEQ ID NO: 9
ART DER SEQUENZ: Nukleotidsequenz
SEQUENZLÄNGE: 429 Nukleotide
ART DES MOLEKÜLS: reifes Protein kodierende cDNA Ti12
URSPRÜNGLICHE HERKUNFT: Speicheldrüse von Triatoma pallidipennis
EIGENSCHAFTEN: kodierende cDNA

GCA GAA GGT GAC GAC TGT TCA TTA GAA AAA GCT ATG GGG GAC TTT 045
AAA CCA GAG GAG TTT TTC AAT GGA ACG TGG TAT TTG GCT CAT GGA 090
CCG GGC GTA ACA AGT CCA GCT GTC TGT CAG AAA TTT ACT ACT AGT 135
GGA AGC AAA GGT TTC ACC CAA ATT GTT GAA ATA GGG TAC AAC AAA 180
TTT GAA AGT AAC GTG AAA TTC CAA TGC AAT CAA GTT GAT AAT AAA 225
AAT GAC GAA CAA TAT TCT TTC AAA TGC AAA GGT AGT GAT AAT ACT 270
GAA TTC GAA GCA GAT TTT ACA TTT ATT AGT GTA AGC TAT GAT AAC 315
TTT GCT TTA GTT TGT AGA AGT ATC ATA TTT ACA TCA CAG CCT AAG 360
GAA GAT GAT TAT TTG GTA TTC GAA CGG ACT AAA AGT GAC ACA GAT 405
CCT GAT GCT AAT GAA ATT TGT TAG 429

SEQ ID NO: 10
ART DER SEQUENZ: Nukleotidsequenz
SEQUENZLÄNGE: 429 Nukleotide
ART DES MOLEKÜLS: reifes Protein kodierende cDNA Ti28
URSPRÜNGLICHE HERKUNFT: Speicheldrüse von Triatoma pallidipennis
EIGENSCHAFTEN: kodierende cDNA

GCA GAA GGT GAC GAC TGT TCA TTA GAA AAA GCT ATG GGG GAC TTT 045
AAA CCA GAG GAG TTT TTC AAT GGA ACG TGG TAT TTG GCT CAT GGA 090
CCG GAC GTA ACA AGT CCA GCT GTC TGT CAG AAA TTT ACT ACT AGT 135
GGA AGC AAA GGT TTC ACC CAA ATT GTT GAA ATA GGG TAC AAC AAA 180
TTT GAA AGT AAC GTG AAA TTC CAA TGC AAT CAA GTT GAT AAT AAA 225
AAT GAC GAA CAA TAT TCT TTC AAA TGC AAA GGT AGT GAT AAT ACT 270
GAA TTC GAA GCA GAT TTT ACA TTT ATT AGT GTA AGC TAT GAT AAC 315
TTT GCT TTA GTT TGT AGA AGT ATC ATA TTT ACA TCA CAG CCT AAG 360
GAA GAT GAT TAT TTG GTA TTC GAA CGG ACT AAA AGT GAC ACA GAT 405
CCT GAT GCT AAT GAA ATT TGT TAG 429

SEQ ID NO: 11
ART DER SEQUENZ: Nukleotidsequenz
SEQUENZLÄNGE: 429 Nukleotide
ART DES MOLEKÜLS: reifes Protein kodierende cDNA Ti45
URSPRÜNGLICHE HERKUNFT: Speicheldrüse von Triatoma pallidipennis
EIGENSCHAFTEN: kodierende cDNA

GCA GAA GGT GAT GAC TGT TCA TTA GAA AAA GCT ATG GGG GAC TTT 045
AAA CCA GAG GAG TTT TTC AAT GGA ACG TGG TAT TTG GCT CAT GGA 090
CCG GAC GTA ACA AGT CCA GCT GTC TGT CAG AAA TTT ACT ACT AGT 135
GGA AGC AAA GGT TTC ACC CAA ATT GTT GAA ATA GGG TAC AAC AAA 180
TTT GAA AGT AAC GTG AAA TTC CAA TGC AAT CAA GCT GAC AAA AAA 225
AAT GAC GAA CAA TAT TCT TTC AAA TGC AAA GGT AGT GAT AAT ACT 270
GAA TTC GAA GCA GAT TTT ACA TTT ATT AGT GTA AGC TAT GAT AAC 315
TTT GCT CTA GTT TGT AGA AGT ATC ACA TTT ACA TCA CAG CCT AAG 360
GAA GAT GAT TAT TTG GTA TTC GAA CGG ACT AAA AGT GAC ACA GAT 405
CCT GAT GCT AAT GAA ATT TGT TAG 429

SEQ ID NO: 12
ART DER SEQUENZ: Nukleotidsequenz
SEQUENZLÄNGE: 429 Nukleotide
ART DES MOLEKÜLS: reifes Protein kodierende cDNA Ti5
URSPRÜNGLICHE HERKUNFT: Speicheldrüse von Triatoma pallidipennis
EIGENSCHAFTEN: kodierende cDNA

GCA GAA GGT GAT GAC TGT TCA ATA GAA AAA GCT ATG GGG GAC TTT 045
AAA CCA GAG GAG TTT TTC AAT GGA ACG TGG TAT TTG GCT CAT GGA 090
CCG GGC GTA ACA AGT CCA GCT GTC TGT CAG AAA TTT ACT ACT AGT 135
GGA AGC AAA GGT TTC ACC CAA ATT GTT GAA ATA GGG TAC AAC AAA 180
TTT GAA AGT AAC GTG AAA TTC CAA TGC AAT CAA GTT GAC AAT AAA 225
AAT GGC GAA CAA TAT TCT TTC AAA TGC AAA AGT AGT GAT AAT ACT 270
GAA TTC GAA GCA GAT TTT ACA TTT ATT AGT GTA AGC TAT GAT AAC 315
TTT GCT CTA GTT TGT AGA AGT ATC ACA TTT ACA TCA CAG CCT AAG 360
GAA GAT GAT TAT TTG GTA TTA GAA CGG ACT AAA AGT GAC ACA GAT 405
CCT GAT GCT AAA GAA ATT TGT TAG 429

SEQ ID NO: 13
ART DER SEQUENZ: Nukelotidsequenz
SEQUENZLÄNGE: 483 Nukleotide
ART DES MOLEKÜLS: reifes Protein mit Signalsequenz kodierende cDNA
Ti12
URSPRÜNGLICHE HERKUNFT: Speicheldrüse von Triatoma pallidipennis
EIGENSCHAFTEN: kodierende cDNA

ATG AAG ACG ATC ATT GCA GTG ACA ATT TTT GGA ATT TTG ACA TGT 045
GCA TAT GCA GCA GAA GGT GAC GAC TGT TCA TTA GAA AAA GCT ATG 090
GGG GAC TTT AAA CCA GAG GAG TTT TTC AAT GGA ACG TGG TAT TTG 135
GCT CAT GGA CCG GGC GTA ACA AGT CCA GCT GTC TGT CAG AAA TTT 180
ACT ACT AGT GGA AGC AAA GGT TTC ACC CAA ATT GTT GAA ATA GGG 225
TAC AAC AAA TTT GAA AGT AAC GTG AAA TTC CAA TGC AAT CAA GTT 270
GAT AAT AAA AAT GAC GAA CAA TAT TCT TTC AAA TGC AAA GGT AGT 315
GAT AAT ACT GAA TTC GAA GCA GAT TTT ACA TTT ATT AGT GTA AGC 360
TAT GAT AAC TTT GCT TTA GTT TGT AGA AGT ATC ATA TTT ACA TCA 405
CAG CCT AAG GAA GAT GAT TAT TTG GTA TTC GAA CGG ACT AAA AGT 450
GAC ACA GAT CCT GAT GCT AAT GAA ATT TGT TAG 483

SEQ ID NO: 14
ART DER SEQUENZ: Nukleotidsequenz
SEQUENZLÄNGE: 483 Nukleotide
ART DES MOLEKÜLS: reifes Protein mit Signalsequenz kodierende
cDNA Ti28
URSPRÜNGLICHE HERKUNFT: Speicheldrüse von Triatoma pallidipennis
EIGENSCHAFTEN: kodierende Sequenz

ATG AAG ACG ATC ATT GCA GTG ACA ATT TTT GGA ATT TTG ACA TGT 045
GCA TAT GCA GCA GAA GGT GAC GAC TGT TCA TTA GAA AAA GCT ATG 090
GGG GAC TTT AAA CCA GAG GAG TTT TTC AAT GGA ACG TGG TAT TTG 135
GCT CAT GGA CCG GAC GTA ACA AGT CCA GCT GTC TGT CAG AAA TTT 180
ACT ACT AGT GGA AGC AAA GGT TTC ACC CAA ATT GTT GAA ATA GGG 225
TAC AAC AAA TTT GAA AGT AAC GTG AAA TTC CAA TGC AAT CAA GTT 270
GAT AAT AAA AAT GAC GAA CAA TAT TCT TTC AAA TGC AAA GGT AGT 315
GAT AAT ACT GAA TTC GAA GCA GAT TTT ACA TTT ATT AGT GTA AGC 360
TAT GAT AAC TTT GCT TTA GTT TGT AGA AGT ATC ATA TTT ACA TCA 405
CAG CCT AAG GAA GAT GAT TAT TTG GTA TTC GAA CGG ACT AAA AGT 450
GAC ACA GAT CCT GAT GCT AAT GAA ATT TGT TAG 483

SEQ ID NO: 15
ART DER SEQUENZ:	Nukleotidsequenz
SEQUENZLÄNGE:	483 Nukleotide
ART DES MOLEKÜLS:	reifes Protein mit Signalsequenz kodierende
	cDNA Ti45
URSPRÜNGLICHE HERKUNFT:	Speicheldrüse von Triatoma pallidipennis
EIGENSCHAFTEN:	kodierende Sequenzr

ATG AAG ACG ATC ATT GCA GTG ACA ATT TTT GGA ATT TTG ACA TGT 045
GCA TAT GCA GCA GAA GGT GAT GAC TGT TCA TTA GAA AAA GCT ATG 090
GGG GAC TTT AAA CCA GAG GAG TTT TTC AAT GGA ACG TGG TAT TTG 135
GCT CAT GGA CCG GAC GTA ACA AGT CCA GCT GTC TGT CAG AAA TTT 180
ACT ACT AGT GGA AGC AAA GGT TTC ACC CAA ATT GTT GAA ATA GGG 225
TAC AAC AAA TTT GAA AGT AAC GTG AAA TTC CAA TGC AAT CAA GCT 270
GAC AAA AAA AAT GAC GAA CAA TAT TCT TTC AAA TGC AAA GGT AGT 315
GAT AAT ACT GAA TTC GAA GCA GAT TTT ACA TTT ATT AGT GTA AGC 360
TAT GAT AAC TTT GCT CTA GTT TGT AGA AGT ATC ACA TTT ACA TCA 405
CAG CCT AAG GAA GAT GAT TAT TTG GTA TTC GAA CGG ACT AAA AGT 450
GAC ACA GAT CCT GAT GCT AAT GAA ATT TGT TAG 483

SEQ ID NO: 16
ART DER SEQUENZ: Nukleotidsequenz
SEQUENZLÄNGE: 483 Nukleotide
ART DES MOLEKÜLS: das reife Protein mit Signalsequenz kodierende
cDNA Ti5
URSPRÜNGLICHE HERKUNFT: Speicheldrüse von Triatoma pallidipennis
EIGENSCHAFTEN: kodierende cDNA

ATG AAG ACG ATC ATT GCA GTG ACA ATT TTT GGA ATT TTG ACA TGT 045
GCA TAT GCA GCA GAA GGT GAT GAC TGT TCA ATA GAA AAA GCT ATG 090
GGG GAC TTT AAA CCA GAG GAG TTT TTC AAT GGA ACG TGG TAT TTG 135
GCT CAT GGA CCG GGC GTA ACA AGT CCA GCT GTC TGT CAG AAA TTT 180
ACT ACT AGT GGA AGC AAA GGT TTC ACC CAA ATT GTT GAA ATA GGG 225
TAC AAC AAA TTT GAA AGT AAC GTG AAA TTC CAA TGC AAT CAA GTT 270
GAC AAT AAA AAT GGC GAA CAA TAT TCT TTC AAA TGC AAA AGT AGT 315
GAT AAT ACT GAA TTC GAA GCA GAT TTT ACA TTT ATT AGT GTA AGC 360
TAT GAT AAC TTT GCT CTA GTT TGT AGA AGT ATC ACA TTT ACA TCA 405
CAG CCT AAG GAA GAT GAT TAT TTG GTA TTA GAA CGG ACT AAA AGT 450
GAC ACA GAT CCT GAT GCT AAA GAA ATT TGT TAG 483

SEQ ID NO: 17
ART DER SEQUENZ: Aminosäure-Sequenz
SEQUENZLÄNGE: 15 Aminosäuren
ART DES MOLEKÜLS: N-terminales Proteinfragment
URSPRÜNGLICHE HERKUNFT: Speicheldrüse von Triatoma pallidipennis
EIGENSCHAFTEN: als reifes Protein:Thrombininhibitor

Ala Glu Gly Asp Asp Cys Ser Leu Glu Lys Ala Met Gly Asp Phe
                5                   10                  15

SEQ ID NO: 18
ART DER SEQUENZ: Aminosäure-Sequenz
SEQUENZLÄNGE: 21 Aminosäuren
ART DES MOLEKÜLS: N-terminales Proteinfragment
URSPRÜNGLICHE HERKUNFT: Speicheldrüse von Triatoma pallidipennis
EIGENSCHAFTEN: als reifes Protein: Thrombininhibitor

Ala Glu Gly Asp Asp Cys Ser Leu Glu Lys Ala Met Gly Asp Phe
                5                   10                  15
Xaa Pro Glu Glu Phe Phe
                20

SEQ ID NO: 19
ART DER SEQUENZ: Aminosäure-Sequenz
SEQUENZLÄNGE: 21 Aminosäuren
ART DES MOLEKÜLS: N-terminales Proteinfragment
URSPRÜNGLICHE HERKUNFT: Speicheldrüse von Triatoma pallidipennis
EIGENSCHAFTEN: als reifes Protein: Thrombininhibitor

Ala Glu Gly Asp Asp Cys Ser Leu Glu Lys Ala Met Gly Asp Phe
                5                   10                  15
Lys Pro Glu Glu Phe Phe
                20

SEQ ID NO: 20
ART DER SEQUENZ:      Aminosäure-Sequenz
SEQUENZLÄNGE:      18 Aminosäuren
ART DES MOLEKÜLS:      N-terminales Proteinfragment nach Spaltung mit Lys
      C
URSPRÜNGLICHE HERKUNFT:   Speicheldrüse von Triatoma pallidipennis
EIGENSCHAFTEN:      als reifes Protein: Thrombininhibitor

Ala - Met - Gly - Asp - Phe - Lys  - Pro - Glu - Glu - Phe -
                        5                                10

Phe -  ?  - Gly - Thr - Arg(?)-Tyr - Leu - Ala
                        15

"?" Unbestimmt;   Arg(?) = Mit Unsicherheit

SEQ ID NO: 21
ART DER SEQUENZ:	Aminosäure-Sequenz
SEQUENZLÄNGE:	12 Aminosäuren
ART DES MOLEKÜLS:	N-terminales Proteinfragment nach Spaltung mit Lys
C
URSPRÜNGLICHE HERKUNFT:	Speicheldrüse von Triatoma pallidipennis
EIGENSCHAFTEN:	als reifes Protein: Thrombininhibitor

Gly - Phe - Thr - Gln - Ile - Val - Glu - Ile - Gly - Tyr -
                        5                                10

Asn - Lys -

SEQ ID NO: 22
ART DER SEQUENZ:	Aminosäure-Sequenz
SEQUENZLÄNGE:	8 Aminosäuren
ART DES MOLEKÜLS:	N-terminales Proteinfragment nach Spaltung mit Lys
C
URSPRÜNGLICHE HERKUNFT:	Speicheldrüse von Triatoma pallidipennis
EIGENSCHAFTEN:	als reifes Protein: Thrombininhibitor

Asn - Gly - Glu - Gln - Tyr - Ser - Phe  Lys -
5

SEQ ID NO: 23
ART DER SEQUENZ: Nukleotidsequenz
SEQUENZLÄNGE: 44 Nukleotide
ART DES MOLEKÜLS: Matritzen-Startstück / Sense Primer
URSPRÜNGLICHE HERKUNFT: Abgeleitet von der Aminosäure-Sequenz
EIGENSCHAFTEN: Primer

GCI GA(A/G) GGI GA(C/T) GA(C/T) TG(C/T) TCI CTI 24
GA(A/G) AA(A/G) GCI ATG GGI GA(C/T) TT 44

SEQ ID NO: 24
ART DER SEQUENZ:	Nukleotidsequenz
SEQUENZLÄNGE:	32 Nukleotide
ART DES MOLEKÜLS:	Nicht-Matritzen- Startstück (Antisense primer):
URSPRÜNGLICHE HERKUNFT:	Abgeleitet von einer Aminosäure-Sequenz
EIGENSCHAFTEN:	Antisense Primer

TT (G/A)TT (G/A)TA ICC (G/A)AT (T/C)TC IAC (G/A)AT 23
(T/C)TG IGT (G/A)AA 32

SEQ ID NO: 25
ART DER SEQUENZ:	Aminosäuresequenz
SEQUENZLÄNGE:	7 Aminosäuren
ART DES MOLEKÜLS:	Peptidfragment
EIGENSCHAFTEN:	C-terminals Ende des modifizierten Cyclophilins a

Phe Ser Ala Ser Ala Leu Ala
                5

SEQ ID NO: 26
ART DER SEQUENZ: Nukleotidsequenz
SEQUENZLÄNGE: 57 Nukleotide
ART DES MOLEKÜLS: Matritzen- Startstück (Sense primer):
URSPRÜNGLICHE HERKUNFT: Abgeleitet von einer Aminosäure-Sequenz
des modifizierten Cyclophilins a
EIGENSCHAFTEN: Sense Primer
GCGATAACAT GTTCAAAAGC ACCCTGGCGG CGATGGCTGC 40
TGTTTTCGCT CTGTCTG 57

SEQ ID NO: 27
ART DER SEQUENZ: Nukleotidsequenz
SEQUENZLÄNGE: 58 Nukleotide
ART DES MOLEKÜLS: Nicht-Matritzen- Startstück (Antisense primer):
URSPRÜNGLICHE HERKUNFT: Abgeleitet von einer Aminosäure-Sequenz
des modifizierten Cyclophilins a
EIGENSCHAFTEN: Antisense Primer
CGCTATAAGC TTCTGCAGGC TAGCGCGCTC GCGCTGAAAG 40
CAGACACAGT CGAAACAG 58

SEQ ID NO: 28
ART DER SEQUENZ: Nukleotidsequenz
SEQUENZLÄNGE: 28 Nukleotide
ART DES MOLEKÜLS: Matritzen- Startstück (Sense primer):
URSPRÜNGLICHE HERKUNFT: Abgeleitet von einer Aminosäure-Sequenz
des erfindungsgemäßen Proteins
EIGENSCHAFTEN: Sense Primer
GCGATAGCTA GCAGCAGAAG GTGACGAC 28

SEQ ID NO: 29
ART DER SEQUENZ:      Nukleotidsequenz
SEQUENZLÄNGE:      43 Nukleotide
ART DES MOLEKÜLS:      Nicht-Matritzen- Startstück (Antisense primer):
URSPRÜNGLICHE HERKUNFT:   Abgeleitet von einer Aminosäure-Sequenz
                                        des erfindungsgemäßen Proteins
EIGENSCHAFTEN:      Antisense Primer
GCGATAGGAT CCAAGCTTAC TAACAAATTT CATTAGCATC AGG   43

## Patentansprüche

1. Natürliches oder synthetisch herstellbares Protein,
das ein Thrombininhibitor ist und aus dem Speichel von Insekten, die Säugetierblut saugen, isolierbar ist,
a) das als aktives Protein eine N-terminale Sequenz wie folgt besitzt: 
Ala • Glu • Gly • Asp • Asp • Cys • Ser • Leu • Glu • Lys • Ala • Met •
5 10
Gly • Asp • Phe • Lys • Pro • Glu • Glu • Phe • Phe • ....
15 20
 oder
b) das als aktives Protein allelische Modifikationen der zuvor unter a) genannten N-terminalen Aminosäure-Sequenz aufweist, wobei eine oder zwei Aminosäuren der N-terminalen Aminosäure-Sequenz substituiert, deletiert oder insertiert sind, ohne dabei die Aktivität des aktiven Proteins wesentlich zu beeinflussen,
oder
c) das als aktives Protein posttranslationale Modifikationen der N-terminalen Sequenzen unter a) und b) aufweist, die nicht wesentlich die Aktivität des aktiven Proteins beeinflussen.

2. Ein Protein, das ein Thrombininhibitor ist und
d) das als aktives reifes Protein eine der folgenden Sequenzen besitzt:
i) Sequence Identifier No. 1 (Sequenz Protokoll Nr. 1)
ii) Sequence Identifier No. 2 (Sequenz Protokoll Nr. 2)
iii) Sequence Identifier No. 3 (Sequenz Protokoll Nr. 3) und
iv) Sequence Identifer No. 4 (Sequenz Protokoll Nr. 4)
oder
e) das als aktives reifes Protein allelische Modifikationen einer der zuvor unter d) genannten Aminosäure-Sequenzen aufweist, wobei wenigstens eine Aminosäure der Aminosäure-Sequenz substituiert, deletiert oder insertiert ist, ohne dabei die Aktivität des aktiven Proteins wesentlich zu beeinflussen,
oder
f) das als aktives reifes Protein posttranslationale Modifikationen einer der Sequenzen unter d) und e) aufweist, die nicht wesentlich die Aktivität des aktiven Proteins beeinflussen.

3. Ein Protein, bestehend aus einer Signalsequenz und einem reifen Protein nach einem der Ansprüche 1 und 2,
g) wobei das Protein eine der folgenden Sequenzen besitzt:
i) Sequence Identifier No. 5 (Sequenz Protokoll Nr. 5)
ii) Sequence Identifier No. 6 (Sequenz Protokoll Nr. 6)
iii) Sequence Identifier No. 7 (Sequenz Protokoll Nr. 7) und
iv) Sequence Identifer No. 8 (Sequenz Protokoll Nr. 8)
oder
h) wobei das Protein allelische Modifikationen einer der zuvor unter g) genannten Aminosäure-Sequenzen aufweist, wobei wenigstens eine Aminosäure der Aminosäure-Sequenz substituiert, deletiert oder insertiert ist, ohne dabei die Aktivität des reifen aktiven Proteins wesentlich zu beeinflussen,
oder
i) wobei das Protein posttranslationale Modifikationen einer der Sequenzen unter g) und h) aufweist, die nicht wesentlich die Aktivität des aktiven reifen Proteins beeinflussen.

4. Ein Protein nach einem der vorherigen Ansprüche, wobei das Protein ein rekombinantes Protein ist.

5. Ein Protein nach einem der vorherigen Ansprüche, wobei das Protein glykosyliert ist.

6. Eine cDNA oder DNA, die einen reifen Thrombininhibitor kodiert,
aa) wobei die cDNA oder DNA eine der folgenden Aminosäure-Sequenzen kodiert:
i) Sequence Identifier No. 1 (Sequenz Protokoll Nr. 1)
ii) Sequence Identifier No. 2 (Sequenz Protokoll Nr. 2)
iii) Sequence Identifier No. 3 (Sequenz Protokoll Nr. 3) und
iv) Sequence Identifer No. 4 (Sequenz Protokoll Nr. 4)
oder
bb) wobei die cDNA oder DNA allelische Modifikationen einer der Aminosäure-Sequenzen unter aa) kodiert,
worin wenigstens eine Aminosäure der Aminosäure-Sequenz substituiert, deletiert oder insertiert ist, ohne dabei die Aktivität des aktiven Proteins wesentlich zu beeinflussen.

7. Eine cDNA oder DNA, die einen Thrombininhibitor kodiert,
cc) wobei die cDNA oder DNA eine der folgenden Nukleotidsequenzen besitzt:
i) Sequence Identifier No. 9 (Sequenz Protokoll Nr. 9)
ii) Sequence Identifier No. 10 (Sequenz Protokoll Nr. 10)
iii) Sequence Identifier No. 11 (Sequenz Protokoll Nr. 11) und
iv) Sequence Identifer No. 12 (Sequenz Protokoll Nr. 12)
oder
dd) wobei die cDNA oder DNA eine allelische Modifikation einer der Nukleotidsequenzen unter cc) aufweist, wobei wenigstens ein Nukleotid substituiert, deletiert oder insedrtiert ist, ohne dabei die Aktivität des Proteines, das von der allelischen Modifikation der Nukleotidsequenz unter cc) kodiert wird, wesentlich zu beeinflussen.

8. Eine cDNA oder DNA, die einen Thrombininhibitor mit Signalsequenz kodiert,
ee) wobei die cDNA oder DNA eine der folgenden Nukleotidsequenzen besitzt:
i) Sequence Identifier No. 13 (Sequenz Protokoll Nr. 13)
ii) Sequence Identifier No. 14 (Sequenz Protokoll Nr. 14)
iii) Sequence Identifier No. 15 (Sequenz Protokoll Nr. 15) und
iv) Sequence Identifer No. 16 (Sequenz Protokoll Nr. 16)
oder
ff) wobei die cDNA oder DNA eine allelische Modifikation einer der Nukleotidsequenzen unter ee) aufweist, wobei wenigstens ein Nukleotid substituiert, deletiert oder insertiert ist, ohne dabei die Aktivität des reifen Proteines, das einschließlich seiner Signalsequenz von der allelischen Modifikation der Nukleotidsequenz unter ee) kodiert wird, wesentlich zu beeinflussen.

9. Ein Vektor, der eine cDNA oder DNA nach einem der Ansprüche 6 bis 8, weiterhin einen passenden Promotor und gegebenenfalls einen passenden Enhancer enthält.

10. Eine eukaryontische oder prokaryontische Wirtszelle, die mit einem Vektor nach Anspruch 9 transformiert ist.

11. Ein Verfahren zur Herstellung der Proteine nach einem der Ansprüche 1 bis 5 mit folgenden Schritten:
Kultivierung einer Wirtszelle, die mit einem Vektor transformiert ist,
welcher eine cDNA oder DNA nach einem der Ansprüche 6 bis 8 enthält,
und
Isolieren und Reinigen der Proteine.

12. Ein Verfahren zur Reinigung von Proteinen nach einem der Ansprüche 1 bis 5, wobei die Proteine isoliert, über wenigstens eine Säule gereinigt und anschließend eingeengt werden.

13. Ein Verfahren zur Reinigung von Proteinen nach einem der Ansprüche 1 bis 5 und 11 bis 12, wobei das Verfahren aus folgenden Schritten besteht:
Auftragen des Speichels auf eine "Superose 12 HR-Säule" und Eluieren und
erneute Auftragung auf einer CH-aktivierten Sepharose-Säule, an der zuvor Thrombin gekoppelt wurde, und Eluieren.

14. Eines der Proteine oder deren Gemisch nach einem der Ansprüche 1 bis 5 und 11 bis 13 als pharmazeutischer Wirkstoff.

15. Ein Arzneimittel, das eines der Proteine oder deren Gemisch nach einem der Ansprüche 1 bis 5 und 11 bis 13 enthält.

16. Eine pharmazeutische Zusammensetzung, die eines der Proteine oder deren Gemisch nach einem der Ansprüche 1 bis 5 und 11 bis 13 enthält, in Gegenwart von pharmazeutisch verträglichen und annehmbaren Verbindungen und Trägern.

17. Verwendung eines der Proteine oder deren Gemisch nach einem der Ansprüche 1 bis 5 und 11 bis 13 zur Herstellung eines Arzneimittels zur Behandlung von
Thrombosen oder instabiler Angina oder Arteriosklerose, oder zur Prävention eines Wiederverschlusses von Gefäßen nach PTCA/PTA oder zur Verhinderung der Blutgerinnung bei der Hämodialyse.

## Claims

1. Natural or synthetic producable protein which is a thrombin inhibitor and can be isolated from the saliva of insects that suck the blood of mammals,
a) which as active protein has an N-terminal sequence: 
Ala • Glu • Gly • Asp • Asp • Cys • Ser • Leu • Glu • Lys • Ala • Met •
5 10
Gly • Asp • Phe • Lys • Pro • Glu • Glu • Phe • Phe • ....
15 20
 or
b) which as active protein exhibits allelic modifications of the N-terminal amino acid sequence under a), wherein one or two amino acids of the N-terminal amino acid sequence are substituted, deleted, or inserted, without affecting the activity of the active protein significantly, or
c) which as active protein exhibits posttranslational modifications of the N-terminal sequences under a) and b), which do not significantly affect the activity of the active protein.

2. A protein which is a thrombin inhibitor and
d) which as active mature protein has one of the following sequences:
i) Sequence identifier No. 1 (sequence protocol No. 1)
ii) Sequence identifier No. 2 (sequence protocol No. 2)
iii) Sequence identifier No. 3 (sequence protocol No. 3) and
iv) Sequence identifier No. 4 (sequence protocol No. 4)
or
e) which as active mature protein exhibits allelic modifications of one of the amino acid sequences mentioned under d), wherein at least one amino acid of the amino acid sequence is substituted, deleted or inserted without affecting the activity of the active protein significantly, or
f) which as active mature protein exhibits posttranslational modifications of one of the sequences under d) and e), which do not significantly affect the activity of the active protein.

3. A protein consisting of a signal sequence and a mature protein according to one of claims 1 and 2,
g) wherein the protein has one of the following sequences:
i) Sequence identifier No. 5 (sequence protocol No. 5)
ii) Sequence identifier No. 6 (sequence protocol No. 6)
iii) Sequence identifier No. 7 (sequence protocol No. 7) and
iv) Sequence identifier No. 8 (sequence protocol No. 8)
or
h) wherein the protein exhibits allelic modifications of one of the amino acid sequences previously mentioned under g), wherein at least one amino acid of the amino acid sequence is substituted, deleted or inserted, without affecting the activity of the mature active protein significantly, or
i) wherein the protein has posttranslational modifications of one of the sequences under g) and h) which do not significantly affect the activity of the active mature protein.

4. A protein according to one of the preceding claims wherein the protein is a recombinant protein.

5. A protein according to one of the preceding claims wherein the protein is glycosylated.

6. A cDNA or DNA, which codes a mature thrombin inhibitor,
aa) wherein the cDNA or DNA codes one of the following amino acid sequences:
i) Sequence identifier No. 1 (sequence protocol No. 1)
ii) Sequence identifier No. 2 (sequence protocol No. 2)
iii) Sequence identifier No. 3 (sequence protocol No. 3) and
iv) Sequence identifier No. 4 (sequence protocol No. 4)
or
bb) wherein the cDNA or DNA codes allelic modifications of one of the amino acid sequences under aa), wherein at least one amino acid of the amino acid sequence is substituted, deleted or inserted, without affecting the activity of the active protein significantly.

7. A cDNA or DNA, which codes a thrombin inhibitor,
cc) wherein the cDNA or DNA has one of the following nucleotide sequences:
i) Sequence identifier No. 9 (sequence protocol No. 9)
ii) Sequence identifier No. 10 (sequence protocol No. 10)
iii) Sequence identifier No. 11 (sequence protocol No. 11) and
iv) Sequence identifier No. 12 (sequence protocol No. 12)
or
dd) wherein the cDNA or DNA exhibits an allelic modification of one of the nucleotide sequences under cc), wherein at least one nucleotide is substituted, deleted or inserted, which is coded by the allelic modification of the nucleotide sequence under cc), without affecting the activity of the protein significantly.

8. A cDNA or DNA, which codes a thrombin inhibitor with signal sequence,
ee) wherein the cDNA or DNA has one the following nucleotide sequences:
i) Sequence identifier No. 13 (sequence protocol No. 13)
ii) Sequence identifier No. 14 (sequence protocol No. 14)
iii) Sequence identifier No. 15 (sequence protocol No. 15) and
iv) Sequence identifier No. 16 (sequence protocol No. 16)
or
ff) wherein the cDNA or DNA has an allelic modification of one of the nucleotide sequences under ee) wherein at least one nucleotide is substituted, deleted or inserted, without affecting the activity of the mature protein significantly including its signal sequence which is coded by the allelic modification of the nucleotide sequence under ee).

9. A vector which contains a cDNA or DNA according to one of claims 6 to 8, further a suitable promoter and obtionally a suitable enhancer.

10. An eukaryotic or prokaryotic host cell, which is transformed with a vector according to claim 9.

11. A process for the production of the proteins according to one of claims 1 to 5 with the following steps:
cultivation of a host cell which is transformed with a vector, which contains a cDNA or DNA according to one of claims 6 to 8 and
isolation and purification of the proteins.

12. A process for the purification of proteins according to one of claims 1 to 5 wherein the proteins are isolated, purified on at least one column and concentrated subsequently.

13. A process for the purification of proteins according to one of claims 1 to 5 and 11 to 12, wherein the process consists of the following steps:
application of saliva on a "Superose 12 HR-column" and elution and
renewed application on a CH-aktivated Sepharose column, to which thrombin was previously coupled, and elution.

14. One of the proteins or their mixture according to one of claims 1 to 5 and 11 to 13, as pharmaceutical agent.

15. A pharmaceutical agent which contains one of the proteins or their mixture according to one of claims 1 to 5 and 11 to 13.

16. A pharmaceutical composition which contains one of the proteins or their mixture according to one of claims 1 to 5 and 11 to 13, in the presence of pharmceutically compatible and acceptable compounds and vehicles.

17. Use of one of the proteins or their mixture according to one of claims 1 to 5 and 11 to 13 for the production of a pharmaceutical agent for treatment of thromboses or unstable angina or arteriosclerosis, or for the prevention of a reblockage of vessels after PTCA/PTA or for the prevention of blood clotting in hemodialysis.

## Revendications

1. Protéine naturelle ou synthétique
laquelle protéine est un inhibiteur de thrombine et que l'on peut isoler à partir de la salive des insectes qui se nourissent de sang des mammifères,
(a) qui contient comme protéine active une séquence N-terminale suivante 
Ala • Glu • Gly • Asp • Asp • Cys • Ser • Leu • Glu • Lys • Ala • Met •
5 10
Gly • Asp • Phe • Lys • Pro•Glu • Glu • Phe • Phe • ....
15 20
 ou
b) qui présente en tant que protéine active des modifications alléliques de la séquence N-terminale d'acides aminés citée au point a), au un ou deux acide aminé de la séquence N-terminale étant substitué, délété ou inséré, sans que l'activité de la protéine active soit essentiellement influencée.
ou
c) qui présente en tant que protéine active des modifications post-traductionnelles des séquences N-terminales au point a) et b), qui n'influencent pas essentiellement l'activité de la protéine active.

2. Protéine, laquelle est un inhibiteur de thrombine et
d) qui comporte en tant que protéine active mature une des séquences suivantes :
i) Séquence identificatrice n° 1 (protocole de séquence n° 1)
ii) Séquence identificatrice n° 2 (protocole de séquence n° 2)
iii) Séquence identificatrice n° 3 (protocole de séquence n° 3) et
iv) Séquence identificatrice n° 4 (protocole de séquence n° 4)
ou
e) qui présente en tant que protéine active mature des modifications alléliques de l'une des séquences d'acides aminés précitées au point d), au moins un acide aminé de la séquence d'acides aminés étant substitué délété ou inséré, sans que l'activité de la protéine active soit essentiellement influencée,
ou
f) qui présente en tant que protéine active mature des modifications post-traductionnelles de l'une des séquences indiquées au points d) et e), qui n'influencent pas essentiellement l'activité de la protéine active.

3. Protéine constituée d'une séquence signal et d'une protéine mature selon l'une des revendications 1 et 2,
g) la protéine comportant une des séquences suivantes :
i) Séquence identificatrice n° 5 (protocole de séquence n° 5)
ii) Séquence identificatrice n° 6 (protocole de séquence n° 6)
iii) Séquence identificatrice n° 7 (protocole de séquence n° 7) et
iv) Séquence identificatrice n° 8 (protocole de séquence n° 8)
ou
h) la protéine présentant dès modifications alléliques de l'une des séquences d'acides aminés précitées au point g), au moins un des acides aminés de la séquence d'acides aminés étant substitué, délété ou inséré, sans que l'activité de la protéine active soit essentiellement influencée,
ou
i) la protéine présentant des modifications post-traductionnelles de l'une des séquences citées au points g) et h) qui n'infleunces essentiellement pas l'activité de la protéine active mature.

4. Protéine selon l'une des revendications précédentes, où la protéine est une protéine recombinée.

5. Protéine selon l'une des revendications précédentes, où la protéine est glycosylée.

6. ADNc ou ADN codant pour un inhibiteur de thrombine mature,
aa) l'ADNc ou l'ADN codant pour une des séquences d'acides aminés suivantes :
i) Séquence identificatrice n° 1 (protocole de séquence n° 1)
ii) Séquence identificatrice n° 2 (protocole de séquence n° 2)
iii) Séquence identificatrice n° 3 (protocole de séquence n° 3) et
iv) Séquence identificatrice n° 4 (protocole de séquence n° 4)
ou
bb) l'ADNc ou l'ADN codant pour des modifications alléliques de l'une des séquences d'acides aminés indiquées au point aa),
au moins un acide aminé de la séquence d'acides aminés étant substitué, délété ou inséré, sans que l'activité de la protéine active soit essentiellement influencée.

7. ADNc ou ADN codant pour un inhibiter de thrombine,
cc) l'ADNc ou l'ADN comportant l'une des séquences de nucléotides suivantes :
i) Séquence identificatrice n° 9 (protocole de séquence n° 9)
ii) Séquence identificatrice n° 10 (protocole de séquence n° 10)
iii) Séquence identificatrice n° 11 (protocole de séquence n° 11) et
iv) Séquence identificatrice n° 12 (protocole de séquence n° 12)
ou
dd) l'ADNc ou l'ADN présentant une modification allélique de l'une des séquences de nucléotides indiquées au point cc), au moins l'un des nucléotides étant substitué, délété ou inséré, sans que l'activité de la protéine codée par la modification allélique de la protéine codée par la modification allélique de la séquence de nucléotides s indiquée ou point cc), soit essentiellement influencée.

8. ADNc ou ADN codant pour un inhibiteur de thormbine avec une séquence signal
ee) l'ADNc ou l'ADN comportant l'une des séquences de nucléotides suivantes :
i) Séquence identificatrice n° 13 (protocole de séquence n° 13)
ii) Séquence identificatrice n° 14 (protocole de séquence n° 14)
iii) Séquence identificatrice n° 15 (protocole de séquence n° 15) et
iv) Séquence identificatrice n° 16 (protocole de séquence n° 16),
ou
ff) l'ADNc ou l'ADN présentant une modification allélique de l'une des séquences de nucléotides indiquées au point ee), au moins un nucléotide étant substitué, délété ou inséré, sans que 1' activité de la protéine mature, laquelle, y compris sa séquence signal, est codée par la modification allélique de la séquence de nucléotides indiquée au point ee), soit essentiellement influencée.

9. Vecteur, contenant un ADNc ou ADN selon l'une des revendications 6 à 8, en outre un promoteur adéquat et éventuellement un amplificateur adéquat.

10. Cellule hôte eucaryote ou procaryote transformée par un vecteur de la revendication 9.

11. Procédé pour la préparation de protéines selon l'une des revendications 1 à 5 avec les étapes suivantes :
culture d'une cellule hôte transformée par un vecteur contenant un ADNc ou ADN selon l'une des revendications 6 à 8, et
isolement et purification des protéines.

12. Procédé de purification de protéines selon l'une des revendications 1 à 5, caractérisé en ce que l'on isole les protéines, on purifie sur au moins une colonne et ensuite, on évapore.

13. Procédé de purification de protéines selon l'une des revendications 1 à 5 et 11 à 12, comportant les étapes suivantes :
dépôt de la salive sur une colonne "Superose 12HR" et élution
et
répétition du dépôt sur une colonne de Sépharose-CH activée, à laquelle on a couplé auparavant la thrombine, et élution.

14. Une des protéines ou son mélange selon l'une des revendications 1 à 5 et 11 à 13 en tant que principe actif pharmaceutique.

15. Médicament contenant l'une des protéines ou son mélange selon l'une des revendications 1 à 5 et 11 à 13.

16. Composition pharmaceutique contenant l'une des protéines ou son mélange selon l'une des revendications 1 à 5 et 11 à 13, en présence de composés et de véhicules pharmaceutiquement acceptables et tolérés.

17. Utilisation de l'une des protéines ou son mélange selon l'une des revendications 1 à 5 et 11 à 13 pour la préparation d'un médicament pour le traitement de thromboses ou d'état de mal angineux ou d'artériosclérose, ou pour la prévention d'une réocclusion de vaisseaux après PTCA/PTA ou pour empêcher la coagulation sanguine dans l'hémodialyse.
